(19) Europäisches Patentamt
European Patent Office
Office européen des brevets

(11) EP 2 627 733 B1

(12) # EUROPÄISCHE PATENTSCHRIFT

(45) Veröffentlichungstag und Bekanntmachung des Hinweises auf die Patenterteilung:
**26.10.2016 Patentblatt 2016/43**

(21) Anmeldenummer: **11761004.8**

(22) Anmeldetag: **14.09.2011**

(51) Int Cl.:
***C09K 19/18*** (2006.01) ***C09K 19/30*** (2006.01)
***C09K 19/42*** (2006.01) ***C09K 19/04*** (2006.01)
***C07C 15/14*** (2006.01) ***C07C 25/24*** (2006.01)

(86) Internationale Anmeldenummer:
**PCT/EP2011/004607**

(87) Internationale Veröffentlichungsnummer:
**WO 2012/048774 (19.04.2012 Gazette 2012/16)**

(54) **VERBINDUNGEN FÜR EIN FLÜSSIGKRISTALLINES MEDIUM UND DEREN VERWENDUNG FÜR HOCHFREQUENZBAUTEILE**

COMPOUNDS FOR A LIQUID CRYSTAL MEDIUM AND USE OF SAID COMPOUNDS FOR HIGH-FREQUENCY COMPONENTS

COMPOSÉS POUR UN MILIEU CRISTAL LIQUIDE ET LEUR UTILISATION POUR DES COMPOSANTS HAUTE FRÉQUENCE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.10.2010 DE 102010048375**

(43) Veröffentlichungstag der Anmeldung:
**21.08.2013 Patentblatt 2013/34**

(73) Patentinhaber: **Merck Patent GmbH**
**64293 Darmstadt (DE)**

(72) Erfinder:
- **REIFFENRATH, Volker**
  **64380 Rossdorf (DE)**
- **JASPER, Christian**
  **64283 Darmstadt (DE)**
- **MANABE, Atsutaka**
  **64625 Bensheim (DE)**
- **MONTENEGRO, Elvira**
  **69469 Weinheim (DE)**
- **PAULUTH, Detlef**
  **64372 Ober-Ramstadt (DE)**

(56) Entgegenhaltungen:
**EP-A1- 0 581 272** **EP-A1- 0 968 988**
**DE-A1- 19 907 941** **DE-A1-102004 029 429**

- **HSU C S ET AL: "SYNTHESIS OF LATERALLY SUBSTITUTED BISTOLANE LIQUID CRYSTALS", LIQUID CRYSTALS: AN INTERNATIONAL JOURNAL OF SCIENCE AND TECHNOLOGY, TAYLOR & FRANCIS, GB, Bd. 27, Nr. 2, 1. Februar 2000 (2000-02-01), Seiten 283-287, XP000932257, ISSN: 0267-8292, DOI: 10.1080/026782900203100 in der Anmeldung erwähnt**
- **ZHENLIN ZHANGA ET AL: "Synthesis and properties of highly birefringent liquid crystalline materials: 2,5-bis(5-alkyl-2-butadinylthiophene-yl) styrene monomers", LIQUID CRYSTALS: AN INTERNATIONAL JOURNAL OF SCIENCE AND TECHNOLOGY, TAYLOR & FRANCIS, GB, Bd. 37, Nr. 1, 1. Januar 2010 (2010-01-01) , Seiten 69-76, XP001553170, ISSN: 0267-8292, DOI: 10.1080/02678290903370272**
- **LIU K ET AL: "SYNTHESIS AND CHARACTERIZATION OF NOVEL FLUORINATED BISTOLANE-TYPE LIQUID CRYSTALS", LIQUID CRYSTALS: AN INTERNATIONAL JOURNAL OF SCIENCE AND TECHNOLOGY, TAYLOR & FRANCIS, GB, Bd. 28, Nr. 10, 1. Oktober 2001 (2001-10-01), Seiten 1463-1467, XP001117376, ISSN: 0267-8292, DOI: 10.1080/02678290110068451**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Bemerkungen:

Die Akte enthält technische Angaben, die nach dem Eingang der Anmeldung eingereicht wurden und die nicht in dieser Patentschrift enthalten sind.

## Beschreibung

Die vorliegende Erfindung betrifft 1,4-Diethinylbenzol-Derivate mit Substituenten in 2,3-Position des Benzolrings (vgl. Formel I, Ansprüche), deren Verwendung für Hochfrequenzbauteile, flüssigkristalline Medien enthaltend die Verbindungen und diese Medien enthaltende Hochfrequenzbauteile, insbesondere Antennen, speziell für den Gigahertzbereich. Die flüssigkristallinen Medien dienen beispielsweise zur Phasenschiebung von Mikrowellen für abstimmbare 'phased-array' Antennen.

Flüssigkristalline Medien werden seit längerem in elektrooptischen Anzeigen (Liquid Crystal Displays - LCDs) genutzt, um Informationen anzuzeigen.

1,4-Diethinylbenzolderivate werden in den Druckschriften EP 0968988 A1, DE 19907941 A1, DE 10120024 A1 und JP 08012599 A als flüssigkristalline Komponenten vorgeschlagen. Die spezielle Substitution dort entspricht jedoch nicht dem Substitutionsmuster der im Rahmen dieser Erfindung wiedergegebenen Verbindungen.

In neuerer Zeit werden flüssigkristalline Medien jedoch auch für die Verwendung in Komponenten, bzw. in Bauteilen, für die Mikrowellentechnik vorgeschlagen, wie z.B. in DE 10 2004 029 429 A und in JP 2005-120208 (A).

Eine technisch wertvolle Anwendung der flüssigkristallinen Medien in der Hochfrequenztechnik beruht auf ihrer Eigenschaft, dass sie sich durch eine variable Spannung in ihren dielektrischen Eigenschaften steuern lassen, besonders für den Gigahertzbereich. Somit lassen sich abstimmbare Antennen konstruieren, die keine beweglichen Teile beinhalten (A. Gaebler, A. Moessinger, F. Goelden, et al., "Liquid Crystal-Reconfigurable Antenna Concepts for Space Applications at Microwave and Millimeter Waves," International Journal of Antennas and Propagation, vol. 2009, Article ID 876989, 7 Seiten, 2009. doi:10.1155/2009/876989).

Die Druckschrift A. Penirschke, S. Müller, P. Scheele, C. Weil, M. Wittek, C. Hock und R. Jakoby: "Cavity Perturbation Method for Characterization of Liquid Crystals up to 35 GHz", 34th European Microwave Conference - Amsterdam, 545-548 beschreibt unter anderen die Eigenschaften der bekannten, flüssigkristallinen Einzelsubstanz K15 (Merck KGaA, Deutschland) bei einer Frequenz von 9 GHz.

1-(Phenylethinyl)tolane, nachfolgend auch Bistolanverbindungen genannt, mit einer Alkylsubstitution am zentralen Phenylenring sind dem Fachmann bekannt. Z.B. offenbart die Druckschrift S.-T. Wu, C.-S. Hsu, K.-F. Shyu Appl. Phys. Lett. (1999), 74 (3), 344-346 verschiedene flüssigkristalline Bistolanverbindungen mit einer lateralen Methylgruppe der Formel

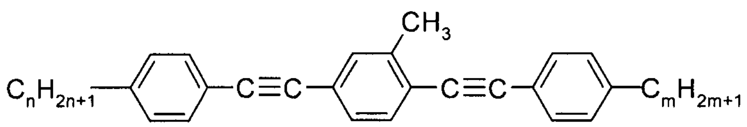

C. S. Hsu, K. F. Shyu, Y.Y. Chuang, S.-T. Wu Liq. Cryst. (2000), 27 (2), 283-287 offenbart, neben solchen flüssigkristallinen Bistolanverbindungen mit einer lateralen Methylgruppe, auch entsprechende Verbindungen mit einer lateralen Ethylgruppe und schlägt deren Verwendung u.a. in "liquid crystal optically phased arrays" vor.

Weitere Bistolanverbindungen werden in den Druckschriften

EP 0581272 A1, Liquid Crystals, 37(1), 69-76 und Liquid Crystals, 28(10), 1463-67 offenbart.

DE 10 2004 029 429 A (vgl. oben) beschreibt die Anwendung von herkömmlichen Flüssigkristallmedien in der Mikrowellentechnik, unter anderem in Phasenschiebern. Dort werden bereits flüssigkristalline Medien bezüglich ihrer Eigenschaften im entsprechenden Frequenzbereich untersucht.

Die bisher bekannten Zusammensetzungen oder Einzelverbindungen sind jedoch in der Regel mit Nachteilen behaftet. Die meisten von ihnen führen, neben anderen Mängeln, zu unvorteilhaft hohen Verlusten und/oder unzureichenden Phasenverschiebungen bzw. zu geringer Materialgüte.

Für die Anwendung in der Hochfrequenztechnik werden flüssigkristalline Medien mit besonderen, bislang eher ungewöhnlichen, ungebräuchlichen Eigenschaften, bzw. Kombinationen von Eigenschaften benötigt.

Somit sind neue Komponenten für flüssigkristalline Medien mit verbesserten Eigenschaften erforderlich. Insbesondere müssen der Verlust im Mikrowellenbereich verringert und die Materialgüte ($\eta$) verbessert werden. Für abstimmbare Antennen werden auch flüssigkristalline Medien mit schneller Reaktionszeit auf eine geänderte Spannung zwischen den Elektroden der Zelle gefordert.

Außerdem besteht der Bedarf das Tieftemperaturverhalten der Bauteile zu verbessern. Hier sind sowohl eine Verbesserung der Betriebseigenschaften, wie auch der Lagerfähigkeit nötig.

Es besteht daher ein erheblicher Bedarf an flüssigkristallinen Medien mit geeigneten Eigenschaften für entsprechende praktische Anwendungen.

Überraschend wurde gefunden, dass die erfindungsgemäßen Verbindungen mit zwei Substituenten in ortho-Position an einem aromatischen Ring, im Vergleich zu einer entsprechenden monosubstituierten Verbindung oder einer disubstituierten Verbindung, bei der die Substituenten nicht in ortho- Position zueinander angeordnet sind, einen deutlich höheren Klärpunkt (Übergang der nematischen Phase in die isotrope Phase) aufweisen. Gleichzeitig ist die Rotationsviskosität ($\gamma_1$) deutlich niedriger als bei den Vergleichsverbindungen mit weniger Substituenten oder ohne ortho-Stellung der Substituenten. Dieser Effekt tritt auf bei Verbindungen, in denen die Ringsysteme durch starre Ethinylenbrücken auf Distanz gehalten werden. Unter Ausnutzung dieses Effekts wurde nun überraschenderweise gefunden, dass mit den erfindungsgemäßen Verbindungen flüssigkristalline Medien mit einem geeigneten, nematischen Phasenbereich und hohem $\Delta n$ verwirklicht werden können, welche die Nachteile der Materialien des Standes der Technik nicht oder zumindest nur in erheblich geringerem Maße aufweisen.

Die Erfindung betrifft Verbindungen der Formel I,

$$R^1\text{-}(A^1\text{-}Z^1)_m\text{—}A^2\text{—}\equiv\text{—}A^3\text{—}\equiv\text{—}(A^4)_k\text{—}(Z^5\text{-}A^5)_n\text{-}R^2 \qquad \text{I}$$

worin

$A^3$

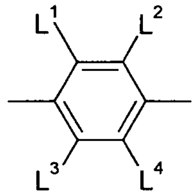

$L^1$, $L^2$ jeweils unabhängig voneinander verzweigtes oder unverzweigtes Alkyl, Alkenyl oder Alkinyl mit 1 bis 12 C-Atomen, worin unabhängig voneinander auch eine oder mehrere "-$CH_2$-"-Gruppen durch O ersetzt sein können, substituiertes oder unsubstituiertes Cycloalkyl, Cycloalkenyl, substituiertes oder unsubstituiertes Phenyl, substituiertes oder unsubstituiertes Arylethinyl, fluoriertes Alkyl oder

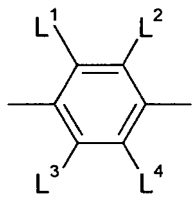

Alkenyl, fluoriertes Alkyloxy oder Alkenyloxy, F, Cl, Br, CN, NCS oder $SF_5$,

$L^3$, $L^4$ H,

$R^1$ und $R^2$ unabhängig voneinander einen halogenierten oder unsubstituierten Alkylrest mit 1 bis 15 C-Atomen, wobei in diesen Resten auch eine oder mehrere $CH_2$-Gruppen jeweils unabhängig voneinander durch -C≡C-, -CH=CH-, -CF=CF-, -CF=CH-, -CH=CF-, -(CO)- und -O- so ersetzt sein können, dass O-Atome nicht direkt miteinander verknüpft sind, F, Cl, Br, CN, $CF_3$, $OCF_3$, NCS oder $SF_5$, $R^2$ auch H,

$A^1$, $A^2$, $A^4$, $A^5$ jeweils unabhängig voneinander

a) 1,4-Phenylen, worin ein oder mehrere, bevorzugt ein bis zwei CH-Gruppen durch N ersetzt sein können,

b) trans-1,4-Cyclohexylen oder Cyclohexenylen, worin auch eine oder zwei nicht benachbarte $CH_2$-Gruppen durch -O- und/oder -S- ersetzt sein können, und worin H durch F ersetzt sein kann,

c) einen Rest der Formeln

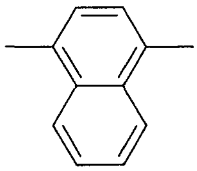

,

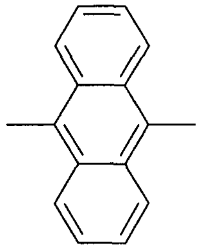

oder

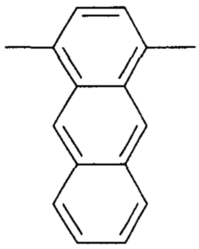

,

oder

d) einen Rest aus der Gruppe 1,4-Bicyclo(2,2,2)-octylen, Cylcobut-1,3-diyl, Spiro[3.3]heptan-2,6-diyl, Thiophen-2,5-diyl, Thiophen-2,4-diyl, Furan-2,5-diyl, Furan-2,4-diyl,

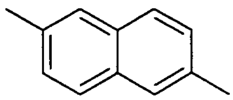

oder

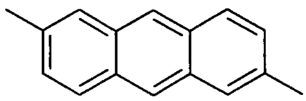

,

und worin in den Gruppen a), b), c) und d)
auch ein oder mehrere H-Atome gegen Br, Cl, F, CN, -NCS, -SCN, $SF_5$, $C_1$-$C_{10}$ Alkyl, $C_1$-$C_{10}$ Alkoxy oder eine ein- oder mehrfach fluorierte $C_1$-$C_{10}$ Alkyl- oder Alkoxygruppe ersetzt sein können, und wobei
mindestens ein Rest aus $A^1$ bis $A^5$, bevorzugt aus $A^2$, $A^3$ und $A^4$, einen Rest nach a) darstellt,

k 0 oder 1,

$Z^1$, $Z^5$ unabhängig voneinander, eine Einfachbindung, -C=C-, -CH=CH-, $-CH_2O-$, -(CO)O-, $-CF_2O-$, $-CF_2CF_2-$, $-CH_2CF_2-$, $-CH_2CH_2-$, $-(CH_2)_4-$, -CH=CF- oder -CF=CF-, wobei asymmetrische Brücken nach beiden Seiten orientiert sein können,
wobei für k = 0 $Z^5$ eine Einfachbindung bedeutet, und m, n unabhängig voneinander 0, 1 oder 2 bedeuten.

Die erfindungsgemäßen Verbindungen besitzen einen hohen Klärpunkt, einen niedrigen Schmelzpunkt, eine außerordentlich hohe optische Anisotropie ($\Delta n$). Schnelle Schaltzeiten werden durch eine überraschend geringe Rotationsviskosität $\gamma_1$ erreicht. Dadurch läst sich ein Phasenschieber schneller justieren. Vorteilhaft ist auch der relativ geringe Verlustfaktor im Mikrowellenspektrum. Die Verbindungen besitzen allein oder in Mischung mit weiteren mesogenen Komponenten über einen breiten Temperaturbereich eine nematische Phase. Diese Eigenschaften machen sie besonders geeignet für die Verwendung in Bauteilen für die Hochfrequenztechnik, insbesondere in flüssigkristallinen Phasenschiebern. Erfindungsgemäße flüssigkristalline Medien besitzen die entsprechenden Eigenschaften, z. B. einen breiten Phasenbereich, schnelle Schaltzeit, und außerdem eine gute Tieftemperaturstabilität.

Bevorzugte Verbindungen der Formel I sind durch die Auswahl eines oder mehrerer der folgenden Parameter gekennzeichnet:

- Der Zähler m ist bevorzugt 0 oder 1, besonders bevorzugt 0. Der Zähler n ist bevorzugt 0 oder 1, besonders bevorzugt 0. m + n ist bevorzugt 0 oder 1.
- Die Ringgruppen $A^1$ und $A^5$ sind unabhängig voneinander bevorzugt ein 1,4-Phenylen, worin auch ein oder mehrere H-Atome gegen Br, Cl, F, CN, Alkyl ($C_1$-$C_{10}$), Methoxy oder eine ein- oder mehrfach fluorierte Methyl- oder Methoxygruppe ersetzt sein können.
- Die Brückengruppen $Z^1$ und $Z^5$ sind unabhängig voneinander bevorzugt eine Einfachbindung, -C≡C-, -CF=CF- oder -CH=CH-, besonders bevorzugt eine Einfachbindung.

Bevorzugt Strukturen sind daher ausgewählt aus I-1 bis I-3:

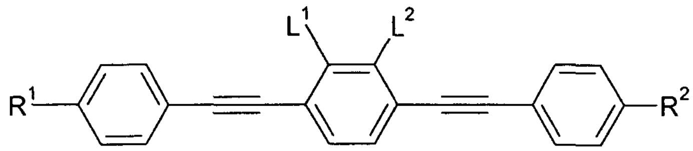

I-1

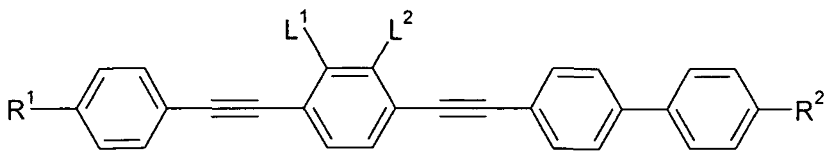

I-2

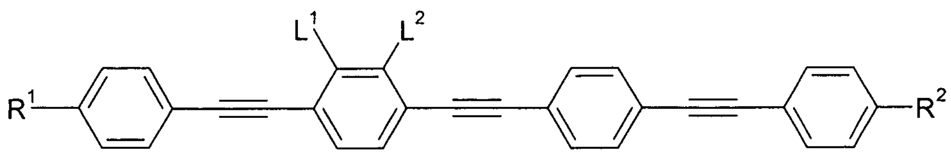

I-3

Einer der Reste $R^1$ oder $R^2$, bevorzugt $R^1$, bedeutet bevorzugt einen geradkettigen Alkylrest mit 1 bis 15 C-Atomen, wobei in diesen Resten auch eine oder mehrere $CH_2$-Gruppen jeweils unabhängig voneinander durch -C≡C-, -CH=CH-, -(CO)O-, -O(CO)-, -(CO)-, -O- so ersetzt sein können, dass O-Atome nicht direkt miteinander verknüpft sind. Bevorzugt sind die Gruppen $R^1$ und $R^2$ beide ein Alkyl mit 2 bis 7 C-Atomen. Dabei bedeuten $R^1$ und $R^2$ beispielsweise Propyl und Hexyl oder Butyl und Butyl, ferner Propyl und Pentyl, Propyl und Hexyl oder Butyl und Pentyl.

Die Gruppen $L^1$ und $L^2$ bedeuten bevorzugt jeweils unabhängig F, Cl, CN, voneinander verzweigtes oder unverzweigtes Alkyl, Alkenyl oder Alkinyl mit 1 bis 8 C-Atomen, worin unabhängig voneinander auch eine oder mehrere "-$CH_2$-"-Gruppen durch O ersetzt sein können, substituiertes oder unsubstituiertes Cycloalkyl, Cycloalkenyl, fluoriertes Alkyl oder Alkenyl, fluoriertes Alkyloxy oder Alkenyloxy mit jeweils bis zu 8 C-Atomen, besonders bevorzugt F, Cl, Alkyl mit 1 bis 5 C-Atomen, Alkenyl mit 2 bis 5 C-Atomen, Cyclopropyl oder Cyclobutyl. Bevorzugt ist eine Gruppe aus $L^{1/2}$ ein F, Methyl, Ethyl, Cyclopropyl oder Cl, besonders bevorzugt F, und die andere Gruppe ist wie oben definiert, oder bevorzugt F, Cl, Alkyl mit 1 bis 5 C-Atomen, Alkenyl mit 2 bis 5 C-Atomen, Cyclopropyl, oder Cyclobutyl.

Die Ringgruppe $A^3$ hat bevorzugt eine Teilstruktur ausgewählt aus den folgenden Formeln:

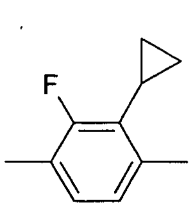 , 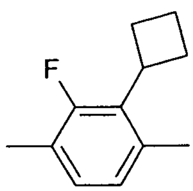 , 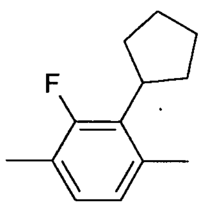 , 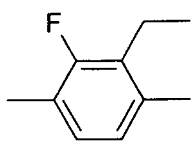 ,

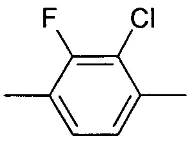 , 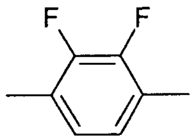 , 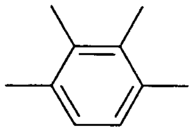 , 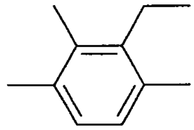 ,

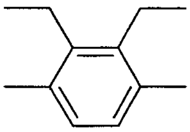 , 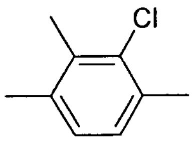 , 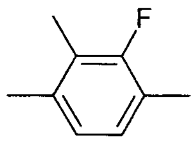 , 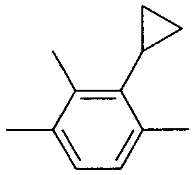 ,

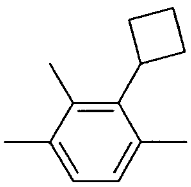 , 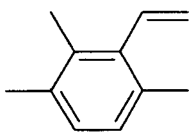 , 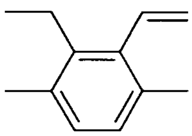

Bevorzugte Ausführungsformen der Erfindung sind daher ausgewählt aus den folgenden beispielhaften Strukturen:

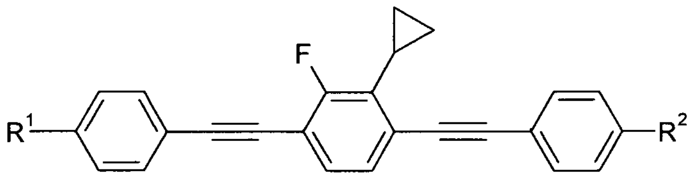

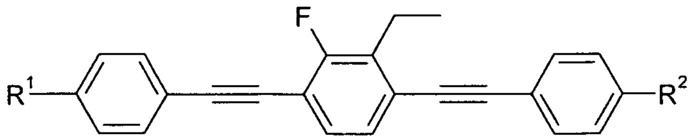

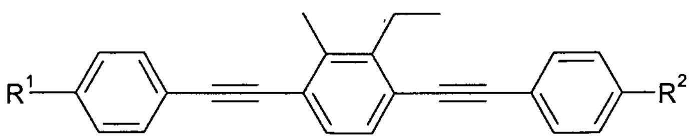

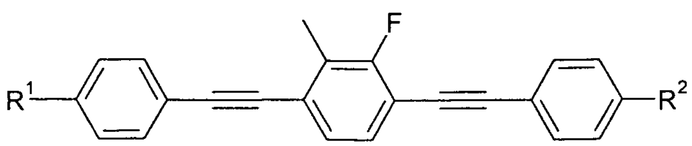

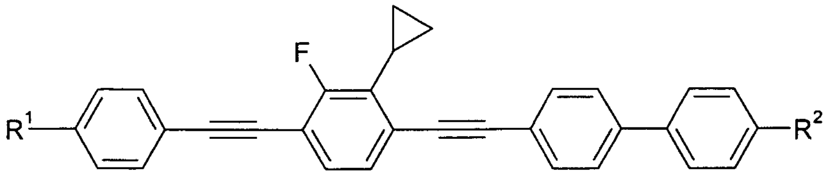

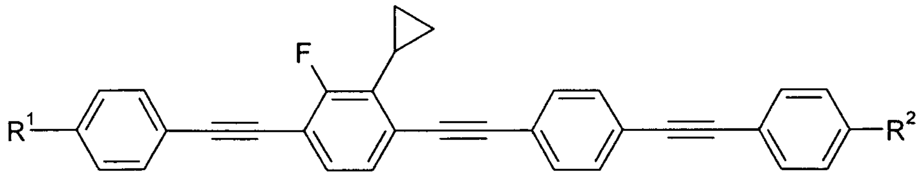

worin $R^1$ und $R^2$ unabhängig einen Alkylrest mit 2 bis 7 C-Atomen, beispielsweise einen Propyl- und Hexylrest oder jeweils einen Propyl-, Butyl-, Pentyl- oder Hexylrest bedeuten.

Für Verbindungen der Formel I, worin k = 0 ist, bedeutet $Z^5$ eine Einfachbindung. Für Verbindungen der Formel I, worin k = 0 ist, nimmt weiterhin die Variable n bevorzugt den Wert 0 an, und der Rest $R^2$ bedeutet bevorzugt einen halogenierten oder unsubstituierten Alkylrest mit 1 bis 15 C-Atomen, wobei in diesem Rest auch eine oder mehrere $CH_2$-Gruppen jeweils unabhängig voneinander durch -C≡C-, -CH=CH-, -CF=CF-, -CF=CH- und -CH=CF- ersetzt sein können, CN, $CF_3$, NCS oder $SF_5$. Besonders bevorzugt bedeutet $R^2$ in diesen Verbindungen einen unsubstituierten Alkylrest mit 1 bis 15 C-Atomen, CN oder NCS.

Die Verbindungen der Formel I können vorteilhaft nach den folgenden allgemeinen Reaktionsschemata (Reaktionsschema I bis VIII) erhalten werden. Die Parameter $R^{1/2}$, $A^{1-5}$, $Z^{1/5}$, m und n sind darin wie vor- und nachstehend definiert. R hat die Bedeutung von $R^{1/2}$.

Reaktionsschema I. $A^2 = A^4$, $Z^5 = Z^1$, $A^5 = A^1$, m = n:

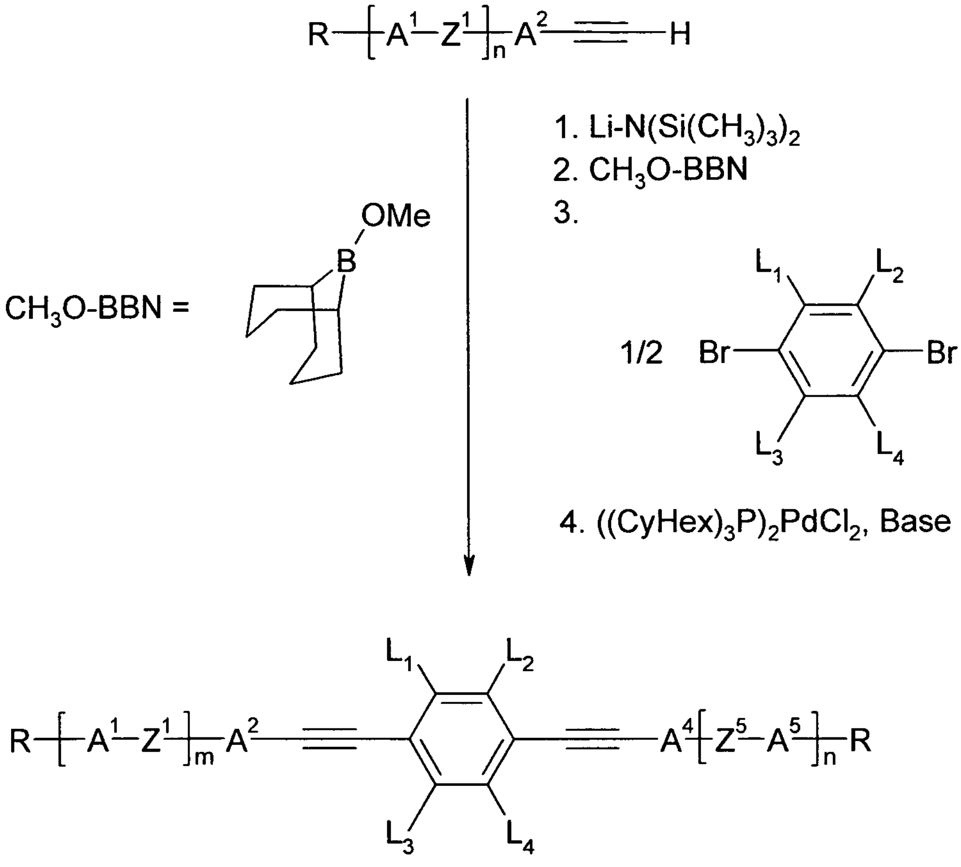

Reaktionsschema II (asymmetrische Substitution):

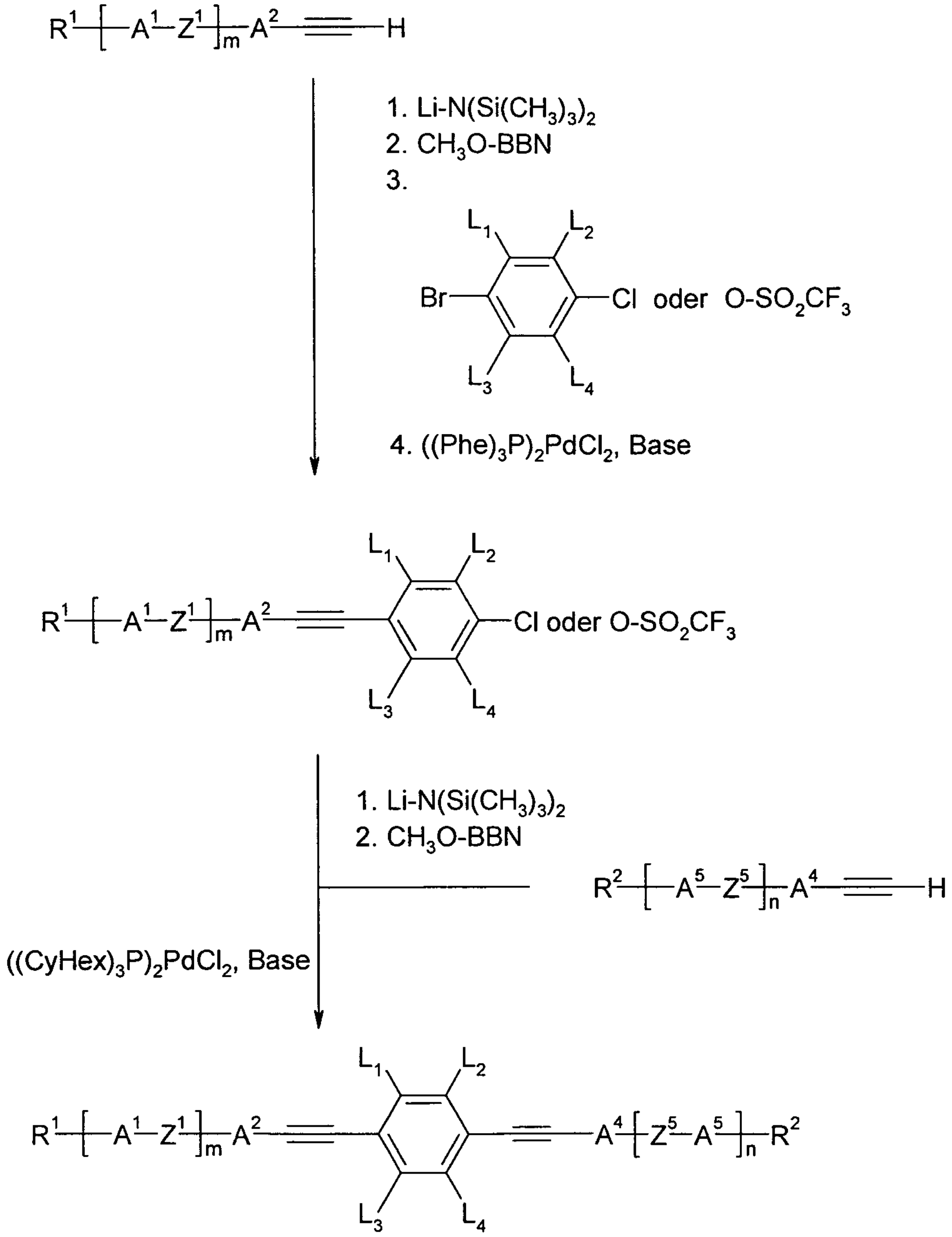


In Reaktionsschema III bis VII wird die Synthese von verschieden substituierten Ringen $A^3$ wiedergegeben. Die Phenylalkinylreste lassen sich dabei auf beliebige Reste $-\equiv-A^2-(Z^1-A^1)_m-R^1$ bzw. $-\equiv-(A^4)_k-(Z^5-A^5)_n-R^2$ verallgemeinern.

Reaktionsschema III (Li-TMP: Li-Tetramethylpiperidid):

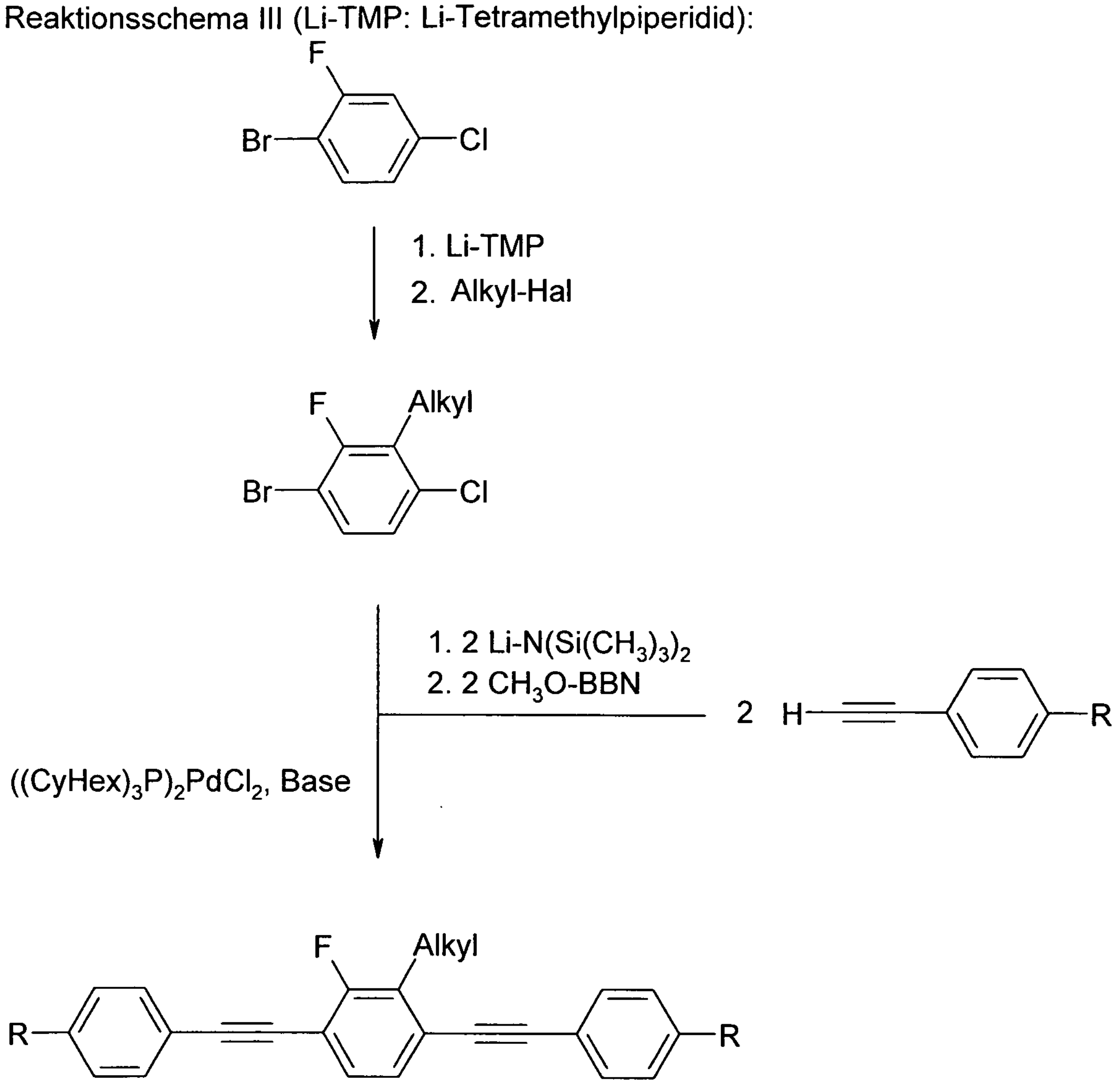

Reaktionsschema IV (LDA: Li-Diisopropylamid):

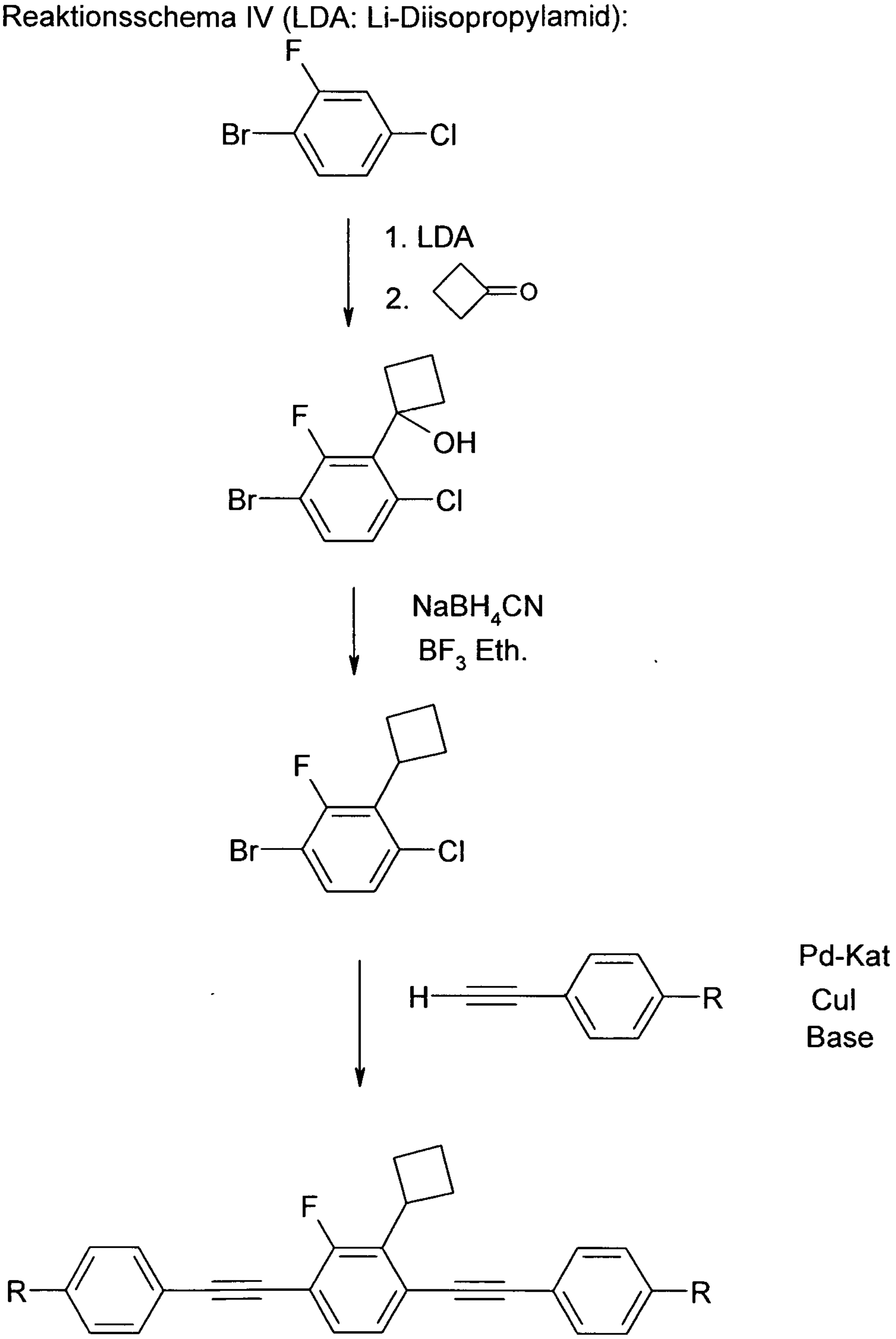

Reaktionsschema V:

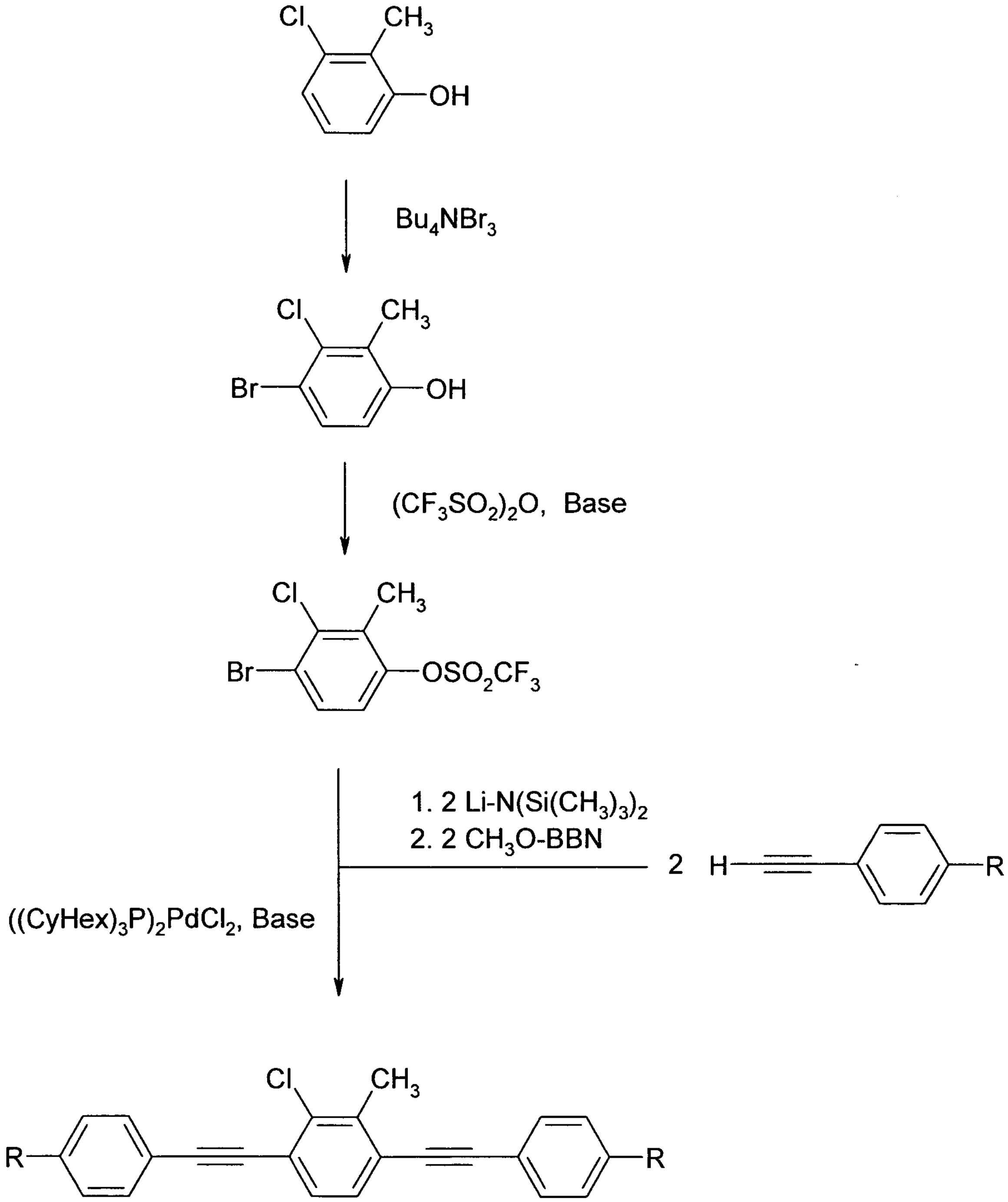

Reaktionsschema VI:

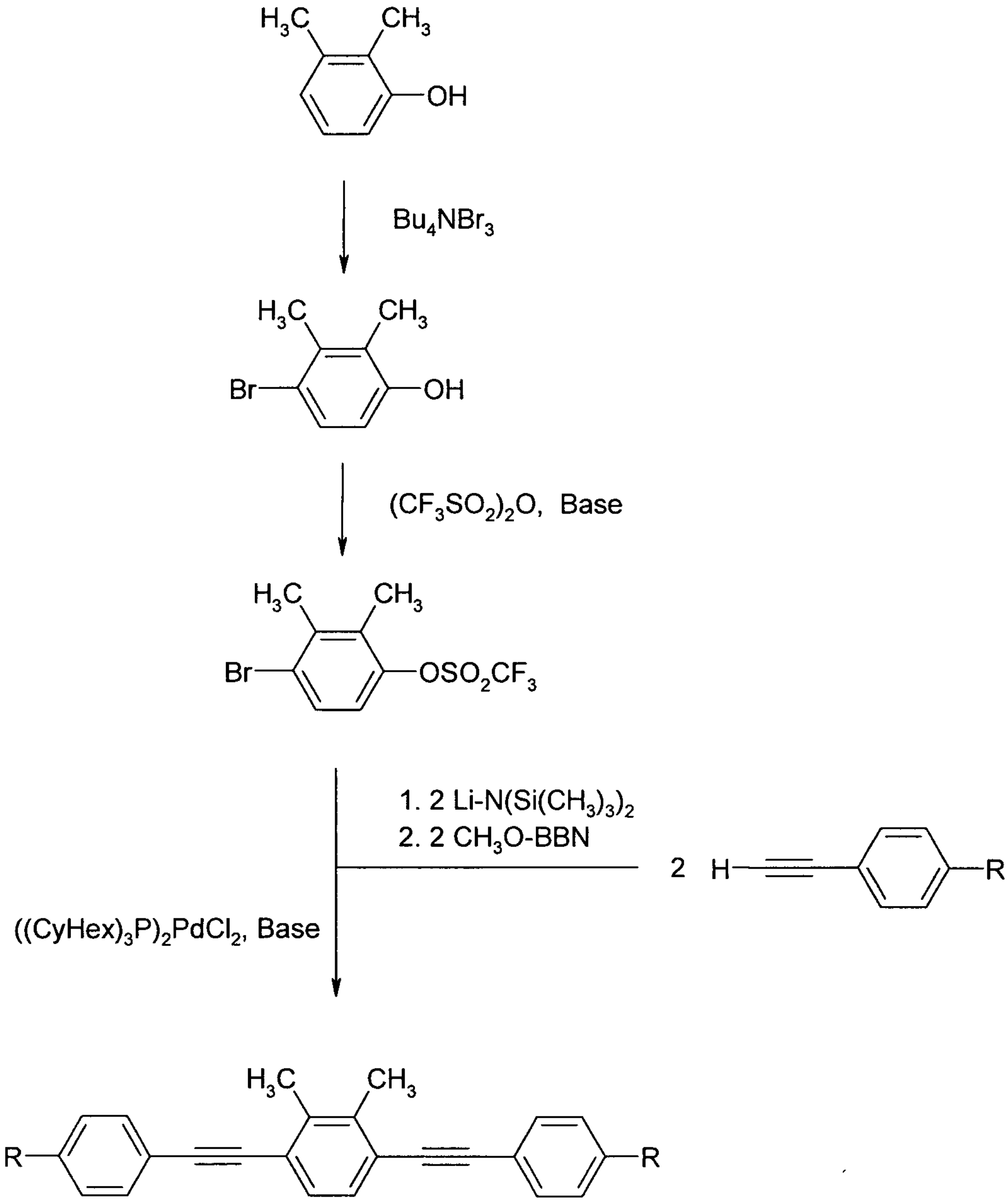

Reaktionsschema VII:

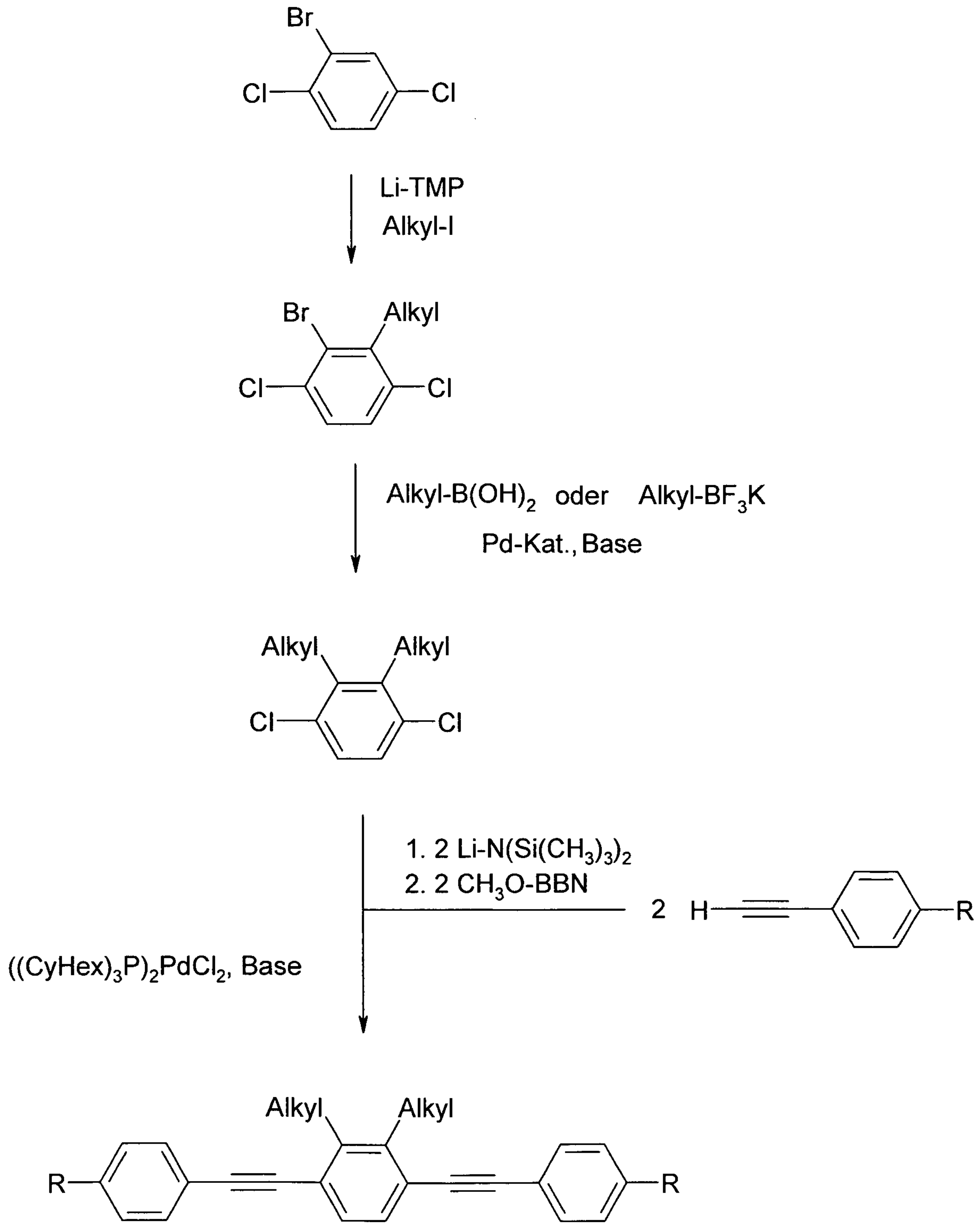

Reaktionsschema VIII (k = 0):

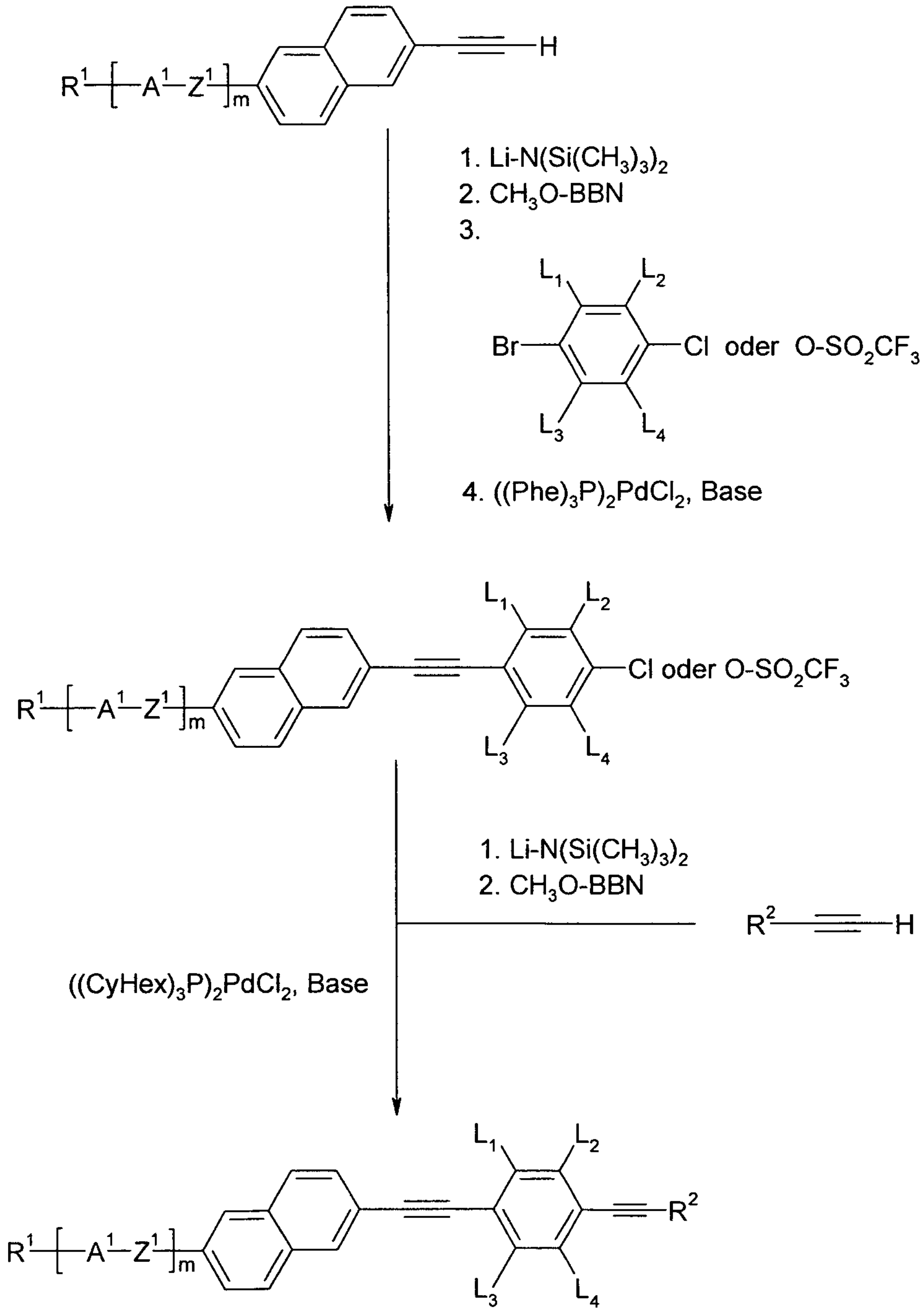


Die flüssigkristallinen Medien gemäß der vorliegenden Erfindung enthalten eine oder mehrere Verbindungen der Formel I und optional mindestens eine weitere, vorzugsweise mesogene Verbindung. Das Flüssigkristallmedium enthält daher bevorzugt zwei oder mehr Verbindungen, die vorzugsweise flüssigkristallin sind. Bevorzugte Medien umfassen die bevorzugten Verbindungen der Formel I.

Weitere Komponenten der flüssigkristallinen Medien sind vorzugsweise ausgewählt aus den Verbindungen der Formel II:

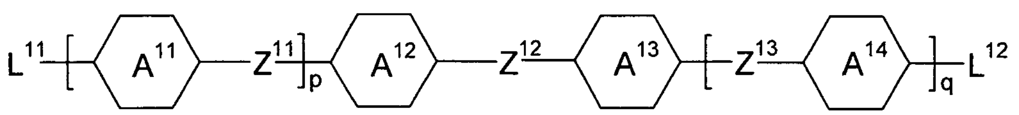 II

worin

| | |
|---|---|
| $L^{11}$ | $R^{11}$ oder $X^{11}$, |
| $L^{12}$ | $R^{12}$ oder $X^{12}$, |
| $R^{11}$ und $R^{12}$ | unabhängig voneinander unfluoriertes Alkyl oder unfluoriertes Alkoxy mit 1 bis 17, bevorzugt mit 3 bis 10, C-Atomen oder unfluoriertes Alkenyl, unfluoriertes Alkinyl, unfluoriertes Alkenyloxy, oder unfluoriertes Alkoxyalkyl mit 2 bis 15, bevorzugt 3 bis 10, C-Atomen, vorzugsweise Alkyl oder unfluoriertes Alkenyl bedeuten, |
| $X^{11}$ und $X^{12}$ | unabhängig voneinander F, Cl, Br, -CN, -NCS, -SCN, -$SF_5$, fluoriertes Alkyl oder fluoriertes Alkoxy mit 1 bis 7 C-Atomen oder fluoriertes Alkenyl, fluoriertes Alkenyloxy oder fluoriertes Alkoxyalkyl mit 2 bis 7 C-Atomen, vorzugsweise fluoriertes Alkoxy, fluoriertes Alkenyloxy, F oder Cl bedeuten, |
| p, q | unabhängig 0 oder 1, |
| $Z^{11}$ bis $Z^{13}$ | unabhängig voneinander *trans*- -CH=CH-, *trans*- -CF=CF-, -C=C- oder eine Einfachbindung bedeuten, und |

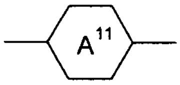

bis

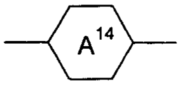

unabhängig voneinander

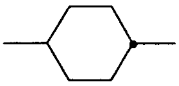 , 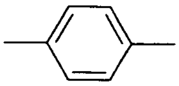 , 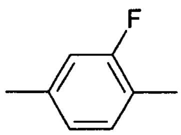 ,

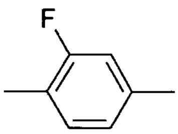 , 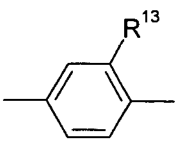 , 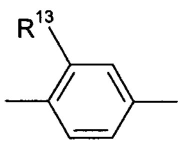 ,

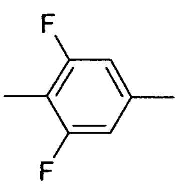 , 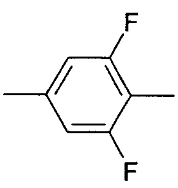 , 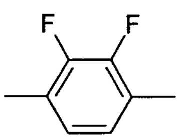 ,

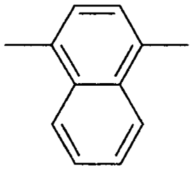,
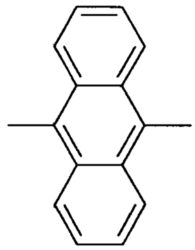

oder

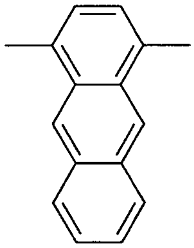,

wobei $R^{13}$ Cl, $C_{1-5}$ Alkyl, $C_{1-5}$ Alkenyl oder $C_{3-6}$ Cycloalkyl bedeutet,

bedeuten.

In einer bevorzugten Ausführungsform der vorliegenden Erfindung enthalten die flüssigkristallinen Medien eine oder mehrere Verbindungen der Formel I und eine oder mehrere Verbindungen der Formel II.

Bevorzugt enthalten die flüssigkristallinen Medien gemäß der vorliegenden Anmeldung insgesamt 5 bis 95 %, bevorzugt 10 bis 90 % und besonders bevorzugt 15 bis 80 %, an Verbindungen der Formel I.

Vorzugsweise enthalten die flüssigkristallinen Medien gemäß der vorliegenden Erfindung Verbindungen ausgewählt aus der Gruppe der Verbindungen der Formeln I und II, stärker bevorzugt bestehen sie überwiegend, noch stärker bevorzugt bestehen sie im Wesentlichen und ganz bevorzugt bestehen sie vollständig daraus.

In dieser Anmeldung bedeutet "enthalten" im Zusammenhang mit Zusammensetzungen, dass die betreffende Entität, d.h. das Medium oder die Komponente, die angegebene Komponente oder Komponenten oder Verbindung oder Verbindungen enthält, vorzugsweise in einer Gesamtkonzentration von 10 % oder mehr und ganz bevorzugt von 20 % oder mehr.

"Überwiegend bestehen" bedeutet in diesem Zusammenhang, dass die betreffende Entität 55 % oder mehr, vorzugsweise 60 % oder mehr und ganz bevorzugt 70 % oder mehr der angegebenen Komponente oder Komponenten oder Verbindung oder Verbindungen enthält.

"Im Wesentlichen" bestehen bedeutet in diesem Zusammenhang, dass die betreffende Entität 80 % oder mehr, vorzugsweise 90 % oder mehr und ganz bevorzugt 95 % oder mehr der angegebenen Komponente oder Komponenten oder Verbindung oder Verbindungen enthält.

"Vollständig bestehen" bedeutet in diesem Zusammenhang, dass die betreffende Entität 98 % oder mehr, vorzugsweise 99 % oder mehr und ganz bevorzugt 100,0 % der angegebenen Komponente oder Komponenten oder Verbindung oder Verbindungen enthält.

Bevorzugt enthalten die flüssigkristallinen Medien gemäß der vorliegenden Anmeldung insgesamt 10 bis 100 %, bevorzugt 20 bis 95 % und besonders bevorzugt 25 bis 90 %, an Verbindungen der Formel I und II.

Gemäß der vorliegenden Erfindung werden die Verbindungen der Formel II vorzugsweise in einer Gesamtkonzentration von 10 % bis 90 %, stärker bevorzugt von 15 % bis 85 %, noch stärker bevorzugt von 25 % bis 80 % und ganz bevorzugt von 30 % bis 75 % der Gesamtmischung verwendet.

Die flüssigkristallinen Medien können darüber hinaus weitere Zusätze wie Stabilisatoren, chirale Dotierstoffe und Nanopartikel enthalten. Die einzelnen, zugesetzten Verbindungen werden in Konzentrationen von 0,005 bis 6 %, vorzugsweise von 0,1 bis 3 %, eingesetzt. Die Gesamtkonzentration dieser weiteren Bestandteile liegt im Bereich von 0 % bis 10 %, vorzugsweise 0,1 % bis 6 %, bezogen auf die Gesamtmischung. Dabei werden jedoch die Konzentrationsangaben der übrigen Bestandteile der Flüssigkristallmischungen, also der flüssigkristallinen oder mesogenen Verbindungen, ohne Berücksichtigung der Konzentration dieser Zusatzstoffe angegeben.

Vorzugsweise enthalten die flüssigkristallinen Medien 0 bis 10 Gew.%, insbesondere 0,01 bis 5 Gew.% und besonders bevorzugt 0,1 bis 3 Gew.% an Stabilisatoren. Vorzugsweise enthalten die Medien einen oder mehrere Stabilisatoren ausgewählt aus 2,6-Di-tert-butylphenolen, 2,2,6,6-Tetramethylpiperidinen oder 2-Benzotriazol-2-yl-phenolen. Diese Hilfsstoffe sind dem Fachmann bekannt und kommerziell erhältlich, z. B. als Lichtschutzmittel.

Eine Ausführungsform der Erfindung ist daher auch ein Verfahren zur Herstellung eines Flüssigkristallmediums, das dadurch gekennzeichnet ist, dass eine oder mehrere Verbindungen der Formel I mit einer oder mehreren weiteren Verbindungen und optional mit einem oder mehreren Additiven gemischt wird. Die weiteren Verbindungen sind vorzugsweise ausgewählt aus den Verbindungen der Formel II, wie oben angegeben, und optional einer oder mehreren weiteren Verbindungen.

In der vorliegenden Anmeldung beschreibt der Ausdruck dielektrisch positiv Verbindungen oder Komponenten mit $\Delta\varepsilon > 3{,}0$, dielektrisch neutral mit $-1{,}5 \leq \Delta\varepsilon \leq 3{,}0$ und dielektrisch negativ mit $\Delta\varepsilon < -1{,}5$. Die dielektrische Anisotropie der jeweiligen Verbindung wird aus den Ergebnissen einer Lösung von 10 % der jeweiligen einzelnen Verbindung in einer nematischen Host-Mischung bestimmt. Wenn die Löslichkeit der jeweiligen Verbindung in der Host-Mischung weniger als 10 % beträgt, wird die Konzentration auf 5 % reduziert. Die Kapazitäten der Testmischungen werden sowohl in einer Zelle mit homeotroper als auch mit homogener Orientierung bestimmt. Die Schichtdicke beträgt bei beiden Zelltypen ca. 20 μm. Die angelegte Spannung ist eine Rechteckwelle mit einer Frequenz von 1 kHz und einem Effektivwert von typischerweise 0,5 V bis 1,0 V, wird jedoch stets so ausgewählt, dass sie unterhalb der kapazitiven Schwelle für die jeweilige Testmischung liegt.

$$\Delta\varepsilon \text{ ist als } (\varepsilon_{||} - \varepsilon_{\perp}) \text{ definiert, während } \varepsilon_{\text{Durchschnitt}} (\varepsilon_{||} + 2\,\varepsilon_{\perp}) / 3 \text{ ist.}$$

Als Host-Mischung wird für dielektrisch positive Verbindungen die Mischung ZLI-4792 und für dielektrisch neutrale sowie für dielektrisch negative Verbindungen die Mischung ZLI-3086 verwendet, beide von Merck KGaA, Deutschland. Die absoluten Werte der dielektrischen Konstanten der Verbindungen werden aus der Änderung der jeweiligen Werte der Host-Mischung bei Zugabe der interessierenden Verbindungen bestimmt. Die Werte werden auf eine Konzentration der interessierenden Verbindungen von 100 % extrapoliert.

Komponenten, die bei der Messtemperatur von 20°C eine nematische Phase aufweisen, werden als solche gemessen, alle anderen werden wie Verbindungen behandelt.

Der Ausdruck Schwellenspannung bezeichnet in der vorliegenden Anmeldung die optische Schwelle und ist für 10 % relativen Kontrast ($V_{10}$) angegeben, der Ausdruck Sättigungsspannung bezeichnet die optische Sättigung und ist für 90 % relativen Kontrast ($V_{90}$) angegeben, in beiden Fällen, soweit nicht ausdrücklich etwas anderes angegeben ist. Die kapazitive Schwellenspannung ($V_0$), auch Freedericks-Schwelle $V_{Fr}$ genannt, wird nur verwendet, wenn dies ausdrücklich genannt ist

Die in dieser Anmeldung angegebenen Parameterbereiche schließen sämtlich die Grenzwerte ein, wenn nicht ausdrücklich etwas anderes angegeben ist.

Die unterschiedlichen für verschiedene Bereiche von Eigenschaften angegebenen oberen und unteren Grenzwerte ergeben in Kombination miteinander zusätzliche bevorzugte Bereiche.

In der gesamten Anmeldung gelten, wenn nicht ausdrücklich anders angegeben, die folgenden Bedingungen und Definitionen. Alle Konzentrationen sind in Massenprozent angegeben und beziehen sich jeweils auf die Gesamtmischung, alle Temperaturen und alle Temperaturunterschiede sind in Grad Celsius bzw. Differenzgrad angegeben. Alle für Flüssigkristalle typischen physikalischen Eigenschaften werden nach "Merck Liquid Crystals, Physical Properties of Liquid Crystals", Stand Nov. 1997, Merck KGaA, Deutschland, bestimmt und sind für eine Temperatur von 20°C aufgeführt, wenn nicht ausdrücklich anders angegeben. Die optische Anisotropie ($\Delta n$) wird bei einer Wellenlänge von 589,3 nm bestimmt. Die dielektrische Anisotropie ($\Delta\varepsilon$) wird bei einer Frequenz von 1 kHz bestimmt. Die Schwellenspannungen sowie alle anderen elektrooptischen Eigenschaften werden mit bei Merck KGaA, Deutschland, hergestellten Testzellen bestimmt. Die Testzellen für die Bestimmung von $\Delta\varepsilon$ besitzen eine Schichtdicke von circa 20 μm. Bei der Elektrode handelt es sich um eine kreisförmige ITO-Elektrode mit einer Fläche von 1,13 $cm^2$ und einem Schutzring. Die Ausrichtungsschichten sind SE-1211 von Nissan Chemicals, Japan, für homeotrope Ausrichtung ($\varepsilon_{\|}$) und Polyimid AL-1054 von Japan Synthetic Rubber, Japan, für homogene Ausrichtung ($\varepsilon_{\perp}$). Die Bestimmung der Kapazitäten erfolgt mit einem Frequenzgang-Analysegerät Solatron 1260 unter Verwendung einer Sinuswelle mit einer Spannung von 0,3 $V_{rms}$. Als Licht wird bei den elektrooptischen Messungen weißes Licht verwendet. Dabei wird ein Aufbau mit einem im Handel erhältlichen Gerät DMS der Fa. Autronic-Melchers, Germany verwendet. Die charakteristischen Spannungen werden unter senkrechter Beobachtung bestimmt. Die Schwellenspannung ($V_{10}$), "Mittgrau-Spannung" ($V_{50}$) und Sättigungsspannung ($V_{90}$) werden für 10 %, 50 % bzw. 90 % relativen Kontrast bestimmt.

Die flüssigkristallinen Medien werden bezüglich ihrer Eigenschaften im Frequenzbereich der Mikrowellen untersucht wie in A. Penirschke et al. "Cavity Perturbation Method for Characterization of Liquid Crystals up to 35GHz", 34th European Microwave Conference - Amsterdam, S. 545-548 beschreiben. Vergleiche hierzu auch A. Gaebler et al. "Direct Simulation of Material Permittivites ...", 12MTC 2009 - International Instrumentation and Measurement Technology Conference, Singapur, 2009 (IEEE), S. 463-467 und DE 10 2004 029 429 A, in der ebenfalls detailliert ein Messverfahren beschrieben wird.

Der Flüssigkristall wird in eine Kapillare aus Polytetrafuorethylen (PTFE) oder Quarzglas gefüllt. Die Kapillare hat einen inneren Radius von 180 $\mu$m und einen äußeren Radius von 350 $\mu$m. Die effektive Länge beträgt 2,0 cm. Die gefüllte Kapillare wird in die Mitte der Kavität mit einer Resonanzfrequenz von 30 GHz eingebracht. Diese Kavität hat eine Länge von 6,6 mm, eine Breite von 7,1 mm und eine Höhe von 3,6 mm. Daraufhin wird das Eingangssignal ("source") angelegt und das Ergebnis des Ausgangssignals mit einem kommerziellen Netzwerkanalysator ("vector network analyzer") aufgenommen. Für andere Frequenzen (z.B. 19 GHz) werden die Abmessungen der Kavität entsprechend angepasst.

Aus der Änderung der Resonanzfrequenz und des Q-Faktors, zwischen der Messung mit der mit dem Flüssigkristall gefüllten Kapillare und der Messung ohne der mit dem Flüssigkristall gefüllten Kapillare, wird die dielektrische Konstante und der Verlustwinkel bei der entsprechenden Zielfrequenz mittels der Gleichungen 10 und 11 der zuvor genannten Druckschrift A. Penirschke et al., 34th European Microwave Conference - Amsterdam, S. 545-548 bestimmt, wie dort beschrieben.

Die Werte für die Komponenten der Eigenschaften senkrecht bzw. and parallel zum Direktor des Flüssigkristalls werden durch Orientierung des Flüssigkristalls in einem Magnetfeld erhalten. Dazu wird das Magnetfeld eines Permanentmagneten verwendet. Die Stärke des Magnetfelds beträgt 0,35 Tesla. Die Orientierung des Magneten wird entsprechend eingestellt und dann entsprechend um 90° gedreht.

Die dielektrische Anisotropie im $\mu$-Wellenbereich ist definiert als

$$\Delta\varepsilon_r \equiv (\varepsilon_{r,||} - \varepsilon_{r,\perp}) .$$

Die Modulierbarkeit bzw. Steuerbarkeit ("tuneability", $\tau$) ist definiert als

$$\tau \equiv (\Delta\varepsilon_r / \varepsilon_{r,||})$$

Die Materialgüte ($\eta$) ist definiert als

$$\eta \equiv (\tau / \tan \delta_{\varepsilon\, r,Max.}) ,$$

mit dem maximalen dielektrischen Verlustfaktor tan $\delta_{\varepsilon\, r,Max}$:

$$\tan \delta_{\varepsilon\, r,Max.} \equiv Max. \{ \tan \delta_{\varepsilon\, r,\perp} ; \tan \delta_{\varepsilon\, r,||} \}$$

der aus dem Maximalwert der gemessenen Werte für tan$\delta_{\varepsilon r}$ hervorgeht.

Die Materialgüte ($\eta$) der bevorzugten Flüssigkristallmaterialien beträgt 6 oder mehr, bevorzugt 8 oder mehr, bevorzugt 10 oder mehr, bevorzugt 15 oder mehr, bevorzugt 17 oder mehr, besonders bevorzugt 20 oder mehr und ganz besonders bevorzugt 25 oder mehr.

Die bevorzugten Flüssigkristallmaterialien haben in den entsprechenden Bauteilen Phasenschiebergüten von 15°/dB oder mehr, bevorzugt von 20°/dB oder mehr, bevorzugt von 30°/dB oder mehr, bevorzugt von 40°/dB oder mehr, bevorzugt von 50°/dB oder mehr, besonders bevorzugt von 80°/dB oder mehr und ganz besonders bevorzugt von 100°/dB oder mehr.

Die erfindungsgemäßen Flüssigkristallmedien weisen bevorzugt nematische Phasen von jeweils mindestens von -20°C bis 80°C, bevorzugt von -30°C bis 85°C und ganz besonders bevorzugt von -40°C bis 100°C auf. Insbesondere bevorzugt reicht die Phase bis 120°C oder mehr, bevorzugt bis 140°C oder mehr und ganz besonders bevorzugt bis 180°C oder mehr. Hierbei bedeutet der Begriff eine nematische Phase aufweisen einerseits, dass bei tiefen Temperaturen bei der entsprechenden Temperatur keine smektische Phase und keine Kristallisation beobachtet wird und andererseits, dass beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen, mit einer Schichtdicke von 5 $\mu$m, für mindestens 100 Stunden überprüft. Bei hohen Temperaturen wird der Klärpunkt nach üblichen Methoden in Kapillaren gemessen.

Die Flüssigkristallmedien gemäß der vorliegenden Erfindung weisen bevorzugt einen Klärpunkt von 90°C oder mehr, stärker bevorzugt von 100°C oder mehr, noch stärker bevorzugt von 120°C oder mehr, besonders bevorzugt von 150°C oder mehr und ganz besonders bevorzugt von 170°C oder mehr, auf.

Das $\Delta\varepsilon$ des Flüssigkristallmediums gemäß der Erfindung bei 1 kHz und 20°C beträgt vorzugsweise 1 oder

mehr, stärker bevorzugt 2 oder mehr und ganz bevorzugt 3 oder mehr.

Das $\Delta n$ der Flüssigkristallmedien gemäß der vorliegenden Erfindung liegt bei 589 nm ($Na^D$) und 20°C vorzugsweise im Bereich von 0,20 oder mehr bis 0,90 oder weniger, stärker bevorzugt im Bereich von 0,25 oder mehr bis 0,90 oder weniger, noch stärker bevorzugt im Bereich von 0,30 oder mehr bis 0,85 oder weniger und ganz besonders bevorzugt im Bereich von 0,35 oder mehr bis 0,80 oder weniger.

In einer bevorzugten Ausführungsform der vorliegenden Anmeldung beträgt das $\Delta n$ der Flüssigkristallmedien gemäß der vorliegenden Erfindung 0,45 oder mehr, vorzugsweise 0,50 oder mehr, und besonders bevorzugt 0,55 oder mehr.

Ferner sind die erfindungsgemäßen Flüssigkristallmedien durch hohe Anisotropien im Mikrowellenbereich gekennzeichnet. Die Doppelbrechung beträgt z.B. bei ca. 8,3 GHz bevorzugt 0,14 oder mehr, besonders bevorzugt 0,15 oder mehr, besonders bevorzugt 0,20 oder mehr, besonders bevorzugt 0,25 oder mehr und ganz besonders bevorzugt 0,30 oder mehr. Außerdem beträgt die Doppelbrechung bevorzugt 0,80 oder weniger.

Die eingesetzten Flüssigkristalle sind entweder Einzelsubstanzen oder Mischungen. Bevorzugt weisen sie eine nematische Phase auf.

In der vorliegenden Anmeldung, bedeutet, wenn nicht ausdrücklich anders angegeben, der Begriff Verbindungen sowohl eine Verbindung, als auch mehrere Verbindungen.

Bevorzugte Bauelemente, die ein Flüssigkristallmedium oder wenigstens eine Verbindung gemäß der Erfindung enthalten, sind Phasenschieber, Varaktoren, Antennenarrays (z. B. für Funk, Mobilfunk, Radio, Mikrowellen/Radar und sonstige Datenübertragung), 'matching circuit adaptive filters' und andere. Bevorzugt sind Bauteile für die Hochfrequenztechnik, wie oben definiert. Bevorzugt sind außerdem durch unterschiedliche angelegte elektrische Spannungen modulierbare Bauteile. Ganz besonders bevorzugte Bauelemente sind Phasenschieber. In bevorzugten Ausführungsformen werden mehrere Phasenschieber funktionell verbunden, wodurch beispielsweise ein phasengesteuerte Gruppenantenne, in der Regel als 'phased array' Antenne bezeichnet, resultiert. Eine Gruppenantenne nutzt die Phasenverschiebung der in einer Matrix angeordneten Sende- oder Empfangselemente, um durch Interferenz eine Bündelung zu erzielen. Aus einer reihen- oder gitterförmigen parallelen Anordnung von Phasenschiebern lässt sich ein sog. 'phased array' aufbauen, das als abstimmbare Sende- oder Empfangsantenne für Hochfrequenzen (z. B. Gigahertzbereich) dienen kann. Erfindungsgemäße 'phased array'-Antennen besitzen einen sehr breiten nutzbaren Empfangskegel.

Bevorzugte Anwendungen sind Radarinstallationen und Datenübertragungsgeräte auf bemannten oder unbemannten Fahrzeugen aus dem Bereich Automobil, Schifffahrt, Flugzeuge, Raumfahrt und Satellitentechnik.

Zur Herstellung geeigneter Bauelemente, insbesondere Phasenschieber, wird typischerweise ein erfindungsgemäßes flüssigkristallines Medium in rechteckige Kavitäten von weniger als 1 mm Dicke, mehreren mm Breite und mehreren Zentimetern Länge eingebracht. Die Kavitäten besitzen entlang zweier langer Seiten angebrachte, gegenüberliegende Elektroden. Solche Anordnungen sind dem Fachmann vertraut. Durch Anlegen einer variablen Spannung können beim Betreiben der Antenne die dielektrischen Eigenschaften des flüssigkristallinen Mediums abgestimmt werden, um verschiedene Frequenzen oder Richtungen einer Antenne einzustellen.

Der Ausdruck "Halogen" oder "halogeniert" steht für F, Cl, Br und I, besonders für F und Cl und insbesondere für F.

Der Ausdruck "Alkyl" umfasst vorzugsweise geradkettige und verzweigte Alkylgruppen mit 1 bis 15 Kohlenstoffatomen, insbesondere die geradkettigen Gruppen Methyl, Ethyl, Propyl, Butyl, Pentyl, Hexyl und Heptyl. Gruppen mit 2 bis 10 Kohlenstoffatomen sind im Allgemeinen bevorzugt.

Der Ausdruck "Alkenyl" umfasst vorzugsweise geradkettige und verzweigte Alkenylgruppen mit 2 bis 15 Kohlenstoffatomen, insbesondere die geradkettigen Gruppen. Besonders bevorzugte Alkenylgruppen sind $C_2$ bis $C_7$-1E-Alkenyl, $C_4$ bis $C_7$-3E-Alkenyl, $C_5$ bis $C_7$-4-Alkenyl, $C_6$ bis $C_7$-5-Alkenyl und $C_7$-6-Alkenyl, insbesondere $C_2$ bis $C_7$-1 E-Alkenyl, $C_4$ bis $C_7$-3E-Alkenyl und $C_5$ bis $C_7$-4-Alkenyl. Beispiele weiterer bevorzugter Alkenylgruppen sind Vinyl, 1 E-Propenyl, 1 E-Butenyl, 1 E-Pentenyl, 1 E-Hexenyl, 1 E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Alkoxy" umfasst vorzugsweise geradkettige Reste der Formel $C_nH_{2n+1}$-O-, worin n 1 bis 10 bedeuten. Vorzugsweise ist n 1 bis 6. Bevorzugte Alkoxygruppen sind beispielsweise Methoxy, Ethoxy, n-Propoxy, n-Butoxy, n-Pentoxy, n-Hexoxy, n-Heptoxy, n-Octoxy, n-Nonoxy, n-Decoxy.

Der Ausdruck "Oxaalkyl" bzw. "Alkoxyalkyl" umfasst vorzugsweise geradkettige Reste der Formel $C_nH_{2n+1}$-O-$(CH_2)_m$, worin n und m jeweils unabhängig voneinander 1 bis 10 bedeuten. Vorzugsweise ist n 1 und m 1 bis 6.

Der Ausdruck "fluorierter Alkylrest" umfasst vorzugsweise ein- oder mehrfach fluorierte Reste. Perfluorierte Reste sind eingeschlossen. Besonders bevorzugt sind $CF_3$, $CH_2CF_3$, $CH_2CHF_2$, $CHF_2$, $CH_2F$, $CHFCF_3$ und $CF_2CHFCF_3$.

Der Ausdruck "fluorierter Alkoxyrest" umfasst ein- oder mehrfach fluorierte Reste. Perfluorierte Reste sind bevorzugt. Besonders bevorzugt ist der Rest $OCF_3$.

Der Ausdruck "substituiertes Cycloalkyl" umfasst ein- oder mehrfach durch Alkyl substituiertes Cycloalkyl, insbesondere Alkyl mit 1 bis 8 Kohlenstoffatomen.

Der Ausdruck "substituiertes Phenyl" umfasst ein- oder mehrfach durch eine Gruppe wie $R^1$ definiert substituiertes Phenyl, insbesondere durch F, Cl, Alkyl oder Alkoxy substituiertes Phenyl.

In der vorliegenden Anmeldung bedeutet Hochfrequenztechnik Anwendungen mit Frequenzen im Bereich von 1 MHz bis 10 THz, bevorzugt von 1 GHz bis 3 THz, stärker bevorzugt 2 GHz bis 1 THz, insbesondere bevorzugt von 5 bis 300 GHz. Die Anwendung liegt bevorzugt im Mikrowellenspektrum oder angrenzenden, für die Nachrichtenübertragung geeigneten Bereichen, in denen 'phased array'-Module in Sende- und Empfangsantennen zum Einsatz kommen können.

Die erfindungsgemäßen Flüssigkristallmedien bestehen aus einer oder mehreren Verbindungen, vorzugsweise aus 2 bis 30, stärker bevorzugt aus 3 bis 20 und ganz bevorzugt aus 3 bis 16 Verbindungen. Diese Verbindungen werden auf herkömmliche Weise gemischt. In der Regel wird die gewünschte Menge der in der geringeren Menge verwendeten Verbindung in der in der größeren Menge verwendeten Verbindung gelöst. Liegt die Temperatur über dem Klärpunkt der in der höheren Konzentration verwendeten Verbindung, ist die Vervollständigung des Lösungsvorgangs besonders leicht zu beobachten. Es ist jedoch auch möglich, die Medien auf anderen üblichen Wegen, beispielsweise unter Verwendung von so genannten Vormischungen, bei denen es sich z.B. um homologe oder eutektische Mischungen von Verbindungen handeln kann, oder unter Verwendung von so genannten "Multi-Bottle"-Systemen, deren Bestandteile selbst gebrauchsfertige Mischungen sind, herzustellen.

Alle Temperaturen, wie z.B. der Schmelzpunkt T(K,N) bzw. T(K,S), der Übergang von der smektischen (S) zur nematischen (N) Phase T(S,N) und der Klärpunkt T (N,I) der Flüssigkristalle sind in Grad Celsius angegeben. Alle Temperaturdifferenzen sind in Differenzgraden angegeben.

In der vorliegenden Anmeldung und in den folgenden Beispielen sind die Strukturen der Flüssigkristallverbindungen durch Akronyme angegeben, wobei die Transformation in chemische Formeln gemäß folgender Tabellen A und B erfolgt. Alle Reste $C_nH_{2n+1}$ und $C_mH_{2m+1}$ sind geradkettige Alkylreste mit n bzw. m C-Atomen; n, m und k sind ganze Zahlen und bedeuten vorzugsweise 0, 1, 2, 3, 4, 5, 6, 7, 8, 9, 10, 11 oder 12. Die Codierung gemäß Tabelle B versteht sich von selbst. In Tabelle A ist nur das Akronym für den Grundkörper angegeben. Im Einzelfall folgt getrennt von Akronym für den Grundkörper mit einem Strich ein Code für die Substituenten $R^{1*}$, $R^{2*}$, $L^{1*}$ und $L^{2*}$:

| Code für $R^{1*}$, $R^{2*}$, $L^{1*}$, $L^{2*}$, $L^{3*}$ | $R^{1*}$ | $R^{2*}$ | $L^{1*}$ | $L^{2*}$ |
|---|---|---|---|---|
| nm | $C_nH_{2n+1}$ | $C_mH_{2m+1}$ | H | H |
| nOm | $C_nH_{2n+1}$ | $OC_mH_{2m+1}$ | H | H |
| nO.m | $OC_nH_{2n+1}$ | $C_mH_{2m+1}$ | H | H |
| n | $C_nH_{2n+1}$ | CN | H | H |
| nN.F | $C_nH_{2n+1}$ | CN | F | H |
| nN.F.F | $C_nH_{2n+1}$ | CN | F | F |
| nF | $C_nH_{2n+1}$ | F | H | H |
| nCl | $C_nH_{2n+1}$ | Cl | H | H |
| nOF | $OC_nH_{2n+1}$ | F | H | H |
| nF.F | $C_nH_{2n+1}$ | F | F | H |
| nF.F.F | $C_nH_{2n+1}$ | F | F | F |
| $nOCF_3$ | $C_nH_{2n+1}$ | $OCF_3$ | H | H |
| $nOCF_3$.F | $C_nH_{2n+1}$ | $OCF_3$ | F | H |
| n-Vm | $C_nH_{2n+1}$ | $-CH=CH-C_mH_{2m+1}$ | H | H |
| nV-Vm | $C_nH_{2n+1}-CH=CH-$ | $-CH=CH-C_mH_{2m+1}$ | H | H |

Geeignete Mischungskomponenten finden sich in den Tabellen A und B.

Tabelle A

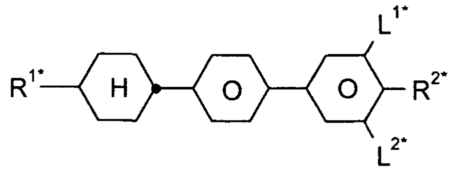

BCH

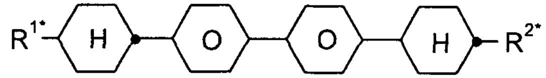

CBC

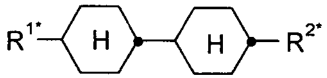

CCH

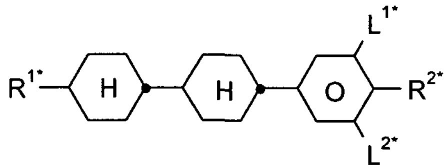

CCP

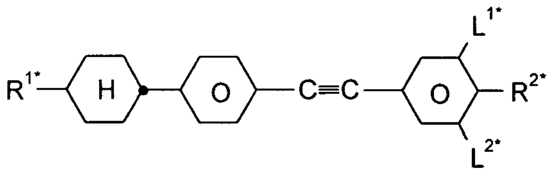

CPTP

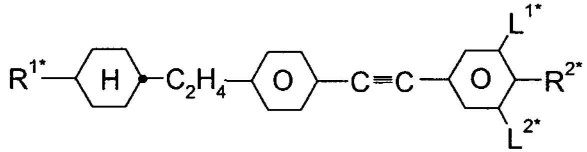

CEPTP

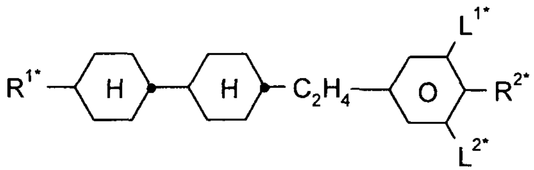

ECCP

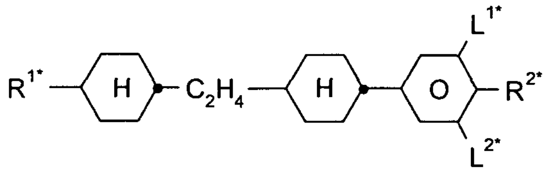

CECP

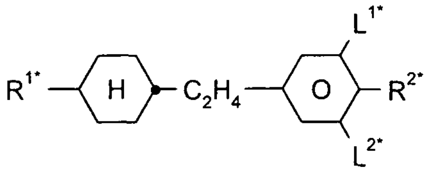

EPCH

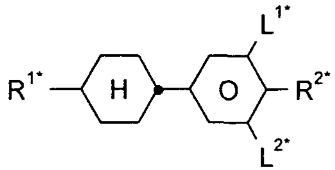

PCH

(fortgesetzt)

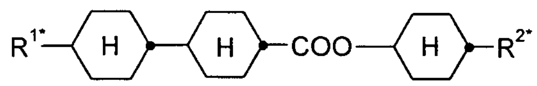

**CH**

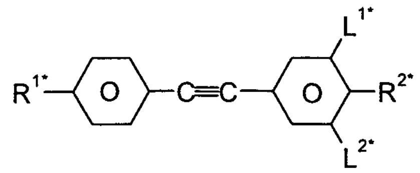

**PTP**

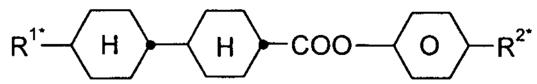

**CP**

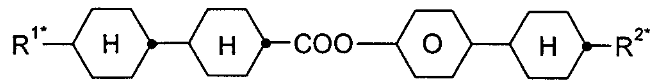

**CCPC**

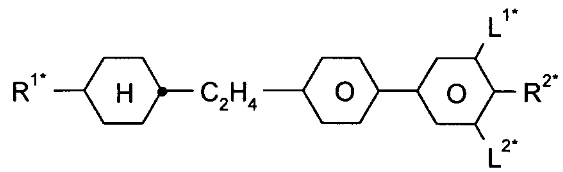

**BECH**

Tabelle B

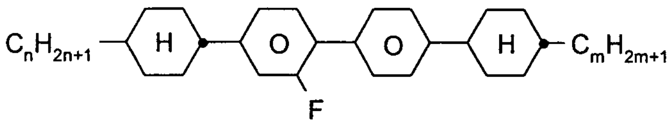

**CBC-nmF**

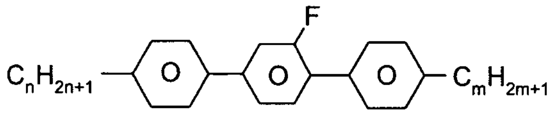

**PGP-n-m**

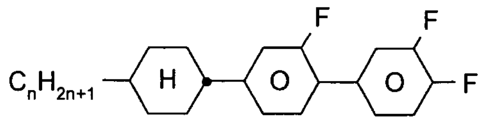

**CGG-n-F**

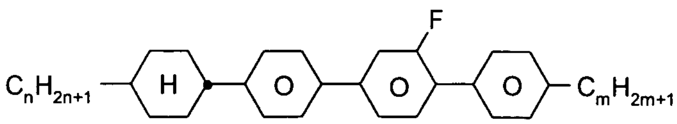

**CPGP-n-m**

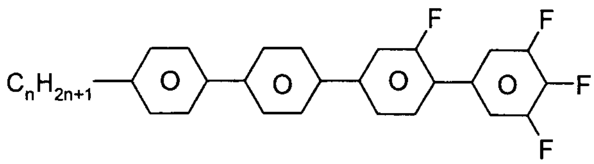

**PPGU-n-F**

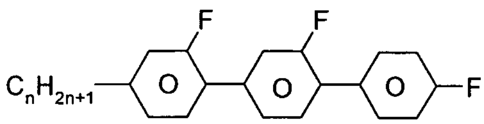

**GGP-n-F**

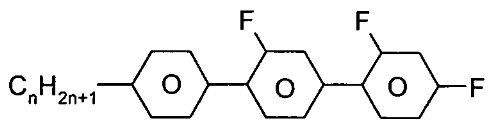

**PGIGI-n-F**

Die folgenden Beispiele erläutern die vorliegende Erfindung, ohne sie in irgendeiner Weise zu beschränken.

Aus den physikalischen Eigenschaften wird dem Fachmann jedoch deutlich, welche Eigenschaften zu erzielen sind und in welchen Bereichen sie modifizierbar sind. Insbesondere ist also die Kombination der verschiedenen Eigenschaften, die vorzugsweise erreicht werden können, für den Fachmann gut definiert.

Verwendete Abkürzungen:

| | |
|---|---|
| DMPU | 1,3-Dimethyl-3,4,5,6-tetrahydro-2(1H)-pyrimidinon |
| DBU | 1,8-Diazabicyclo[5.4.0]undec-7-en bzw. 2,3,4,6,7,8,9,10-Octahydropyrimido[1,2-a]azepin (IUPAC) |

**Beispiele**

Die eingesetzten Acetylene und Boronsäuren sind kommerziell erhältlich oder können in Analogie zu den Schemata I bis VIII oder zu bekannten Synthesen hergestellt werden. Die Reste "$C_4H_9$" stehen für unverzweigte n-Butylreste. Entsprechendes gilt für $C_3H_7$, $C_6H_{13}$, etc..

Synthesebeispiel 1:

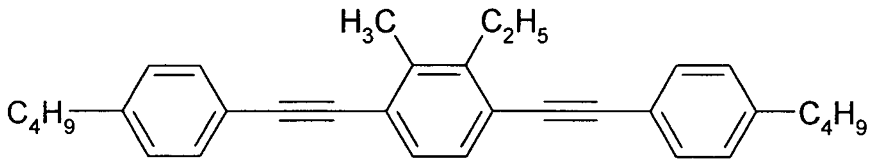

Schritt 1.1

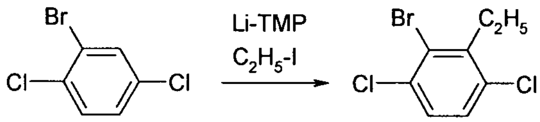


25,4 g 2-Brom-1,4-dichlorbenzol, 8,9 ml Iodethan und 13,3 ml DMPU werden in 230 ml THF gelöst und bei -70°C mit einer Lösung von 16,2 g Lithiumtetramethylpiperidid in THF tropfenweise versetzt. Nach weiteren 2 h bei -70°C lässt man das Reaktionsgemisch auf Umgebungstemperatur erwärmen, hydrolysiert den Ansatz mit Wasser und arbeitet extraktiv auf. Das Rohprodukt wird durch fraktionierte Destillation gereinigt.

Kp : 73°C/0,1 bar. Farblose Flüssigkeit.

Schritt 1.2

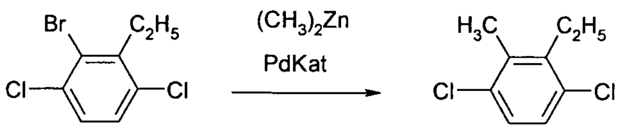


2,4 g wasserfreies Zinkbromid werden in 50 ml THF mit 12,5 ml 5%iger Lösung von Methyllithium in Diethylether bei 25-40°C versetzt. Danach setzt man 0,3 g $PdCl_2$-dppf, Bis(diphenylphosphinoferrocen)-palladiumdichlorid zu, erhitzt zum Sieden und tropft 4,6 g des Produkts des Schritts 1.1, gelöst in etwas THF, zu. Anschließend wird das Reaktionsgemisch für 15 h unter Rückfluss erhitzt. Man hydrolysiert den Ansatz mit Wasser und arbeitet extraktiv auf. Das Rohprodukt wird chromatographisch (Pentan / Kieselgel) aufgereinigt. Farblose Flüssigkeit.

Schritt 1.3

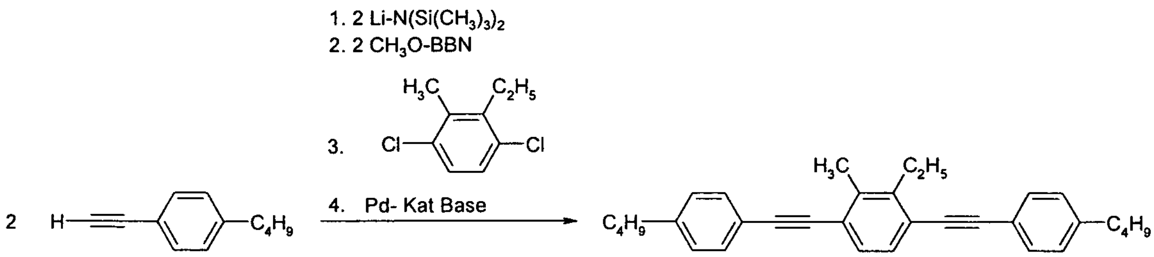


2,4g 4-Butylphenylacetylen werden in 30ml THF vorgelegt und auf-78 °C abgekühlt. Zu dieser Lösung werden 14,3 ml einer 1 M Lösung von Lithium-bis(trimethylsilyl)amid in Hexan zugetropft und bei -78°C 1 h nachreagieren lassen. Danach werden 14,3 ml einer 1 M Lösung von Methoxy-9-BBN zugetropft und 2 h bei -78°C nachgerührt. In einer zweiten Apparatur wurden 1,0g des Produkts des letzten Schritts, gelöst in 40 ml THF, mit dem Katalysator aus 0,2g Tris(dibenzylideneaceton)dipalladium und 0,35 g 2-Dicyclohexylphosphino-2'-6'-dimethoxybiphenyl, vorgelegt, und die Reaktionslösung des ersten Umsatzes bei Raumtemperatur zugesetzt. Es wird 15 h zum Sieden erhitzt. Man hydrolysiert den Ansatz mit Wasser und arbeitet extraktiv auf. Das Rohprodukt wird chromatographisch (Pentan / Kieselgel) aufgereinigt. Nach umkristallisieren aus Pentan erhält man das gereinigte Titelprodukt.

K 45 N 180 I

$$\Delta\varepsilon = +1{,}4$$

$$\Delta n = 0{,}412$$

Vergleichsbeispiel 1

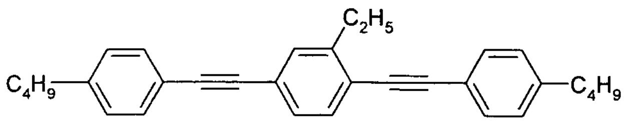

K 29 N 119 I

$$\Delta\varepsilon = +1{,}7$$

$$\Delta n = 0{,}402$$

Synthesebeispiel 2:

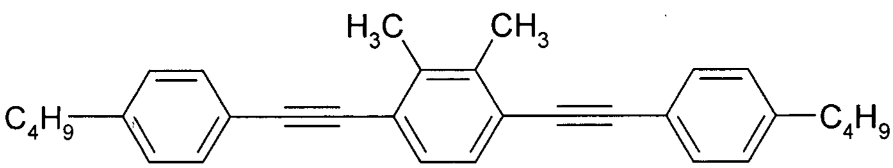

Die Verbindung wird analog zu Beispiel 1 hergestellt.

K 118 N 222 I

$$\Delta\varepsilon = +2{,}6$$

$$\Delta n = 0{,}435$$

$$\gamma_1 = 889\ \mathrm{mPa\cdot s}$$

Svnthesebeispiel 3: (hierin nicht beansprucht)

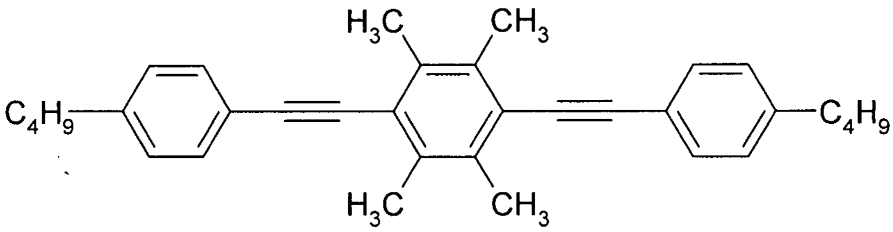

Die Verbindung wird analog zu Beispiel 1 hergestellt.

K 167 N 2251 I

$$\Delta\varepsilon = +2{,}3$$

$$\Delta n = 0{,}423$$

$$\gamma_1 = 1361\ \mathrm{mPa \cdot s}$$

Synthesebeispiel 4:

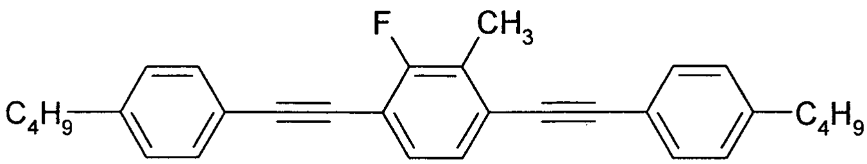

Die Verbindung wird analog zu Beispiel 1 hergestellt.

K 90 N 194 I

$$\Delta\varepsilon = +3{,}0$$

$$\Delta n = 0{,}435$$

$$\gamma_1 = 647\ \mathrm{mPa \cdot s}$$

Vergleichsbeispiel 2:

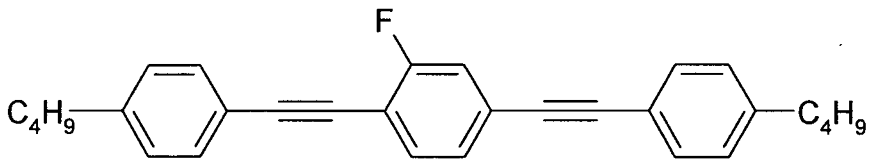

K 93 N 188,5 I

$\Delta\varepsilon$ 4,1

$\Delta n$ 0,452

$\gamma_1$ 374 mPa·s

Synthesebeispiel 5:

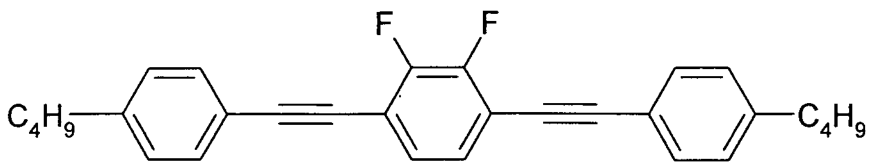

Die Verbindung wird analog zu Beispiel 1 hergestellt.

K 96 N 199 I

$\Delta\varepsilon = -1,4$

$\Delta n = 0,443$

$\gamma_1$ 308 mPa·s

Synthesebeispiel 6:

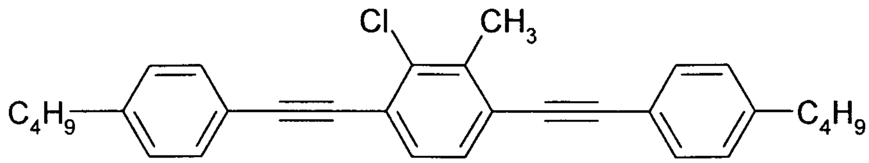

Die Verbindung wird analog zu Beispiel 1 hergestellt.

K 92 N 205 I

$$\Delta\varepsilon = +3,1$$

$$\Delta n = 0,429$$

Synthesebeispiel 7:

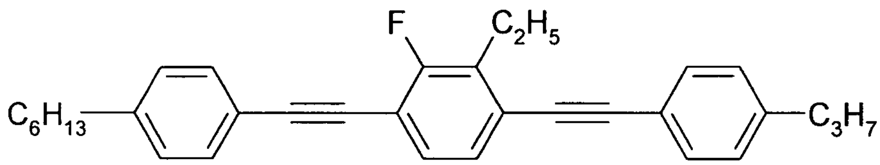

Die Verbindung wird analog zu Beispiel 1 hergestellt.

K 53 N 144 I

$$\Delta\varepsilon = +2,5$$

$$\Delta n = 0,401$$

$$\gamma_1 = 1746 \text{ mPa·s}$$

Vergleichsbeispiel 3:

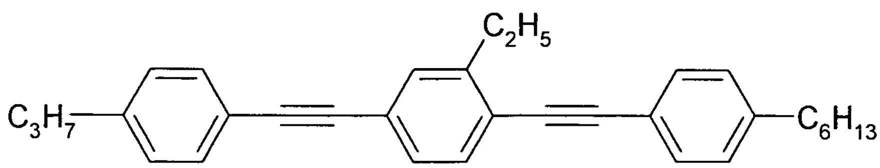

K 60 N 122 I

$$\Delta\varepsilon = +1,7$$

$$\Delta n = 0,394$$

$$\gamma_1 = 1891 \text{ mPa·s}$$

Synthesebeispiel 8:

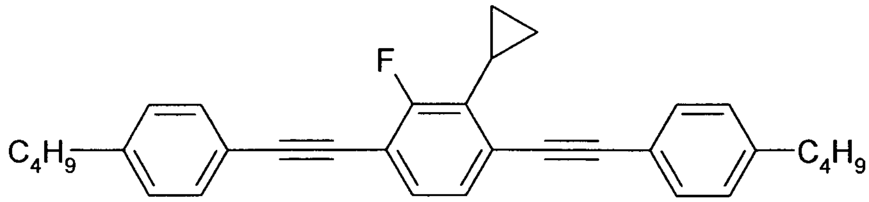

**(1)**

Die Verbindung (1) wird analog zu Beispiel 1 hergestellt und im Mischungsbeispiel 1 verwendet.

TG -49 K 32 N 126 I

$$\Delta\varepsilon = +1,6$$

$$\Delta n = 0,373$$

$$\gamma_1 = 1269 \text{ mPa·s}$$

Vergleichsbeispiel 4:

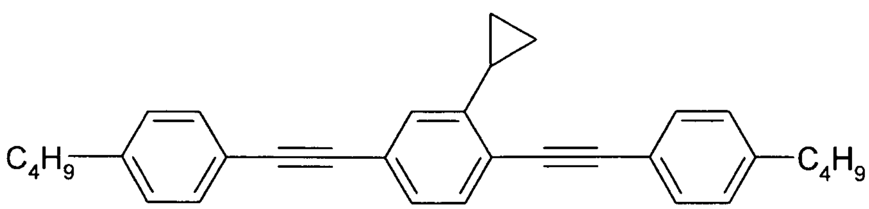

K 72 N 84, 5 I

$$\Delta\varepsilon = +1{,}5$$

$$\Delta n = 0{,}378$$

$$\gamma_1 \ 2194 \ mPa \cdot s$$

Synthesebeispiel 9:

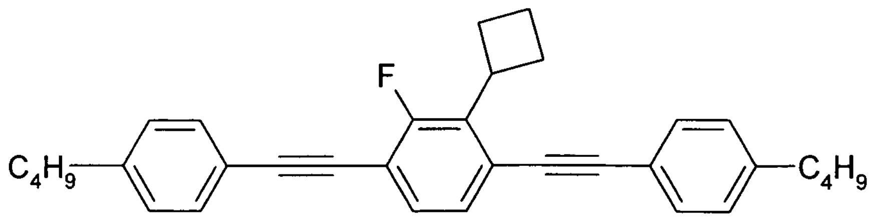

Die Titelverbindung wird analog zu Beispiel 1 hergestellt.

K 60 N 89 I

$$\Delta\varepsilon = +1{,}3$$

$$\Delta n = 0{,}348$$

$$\gamma_1 = 1875 \ mPa \cdot s$$

Vergleichsbeispiel 5:

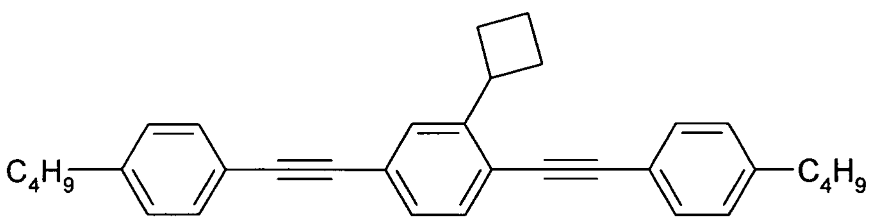

Tg -39 K 69 N 70 I

$$\Delta\varepsilon\ 0{,}9$$

$$\Delta n\ 0{,}359$$

$$\gamma_1\ 3067\ mPa\cdot s$$

Synthesebeispiel 10:

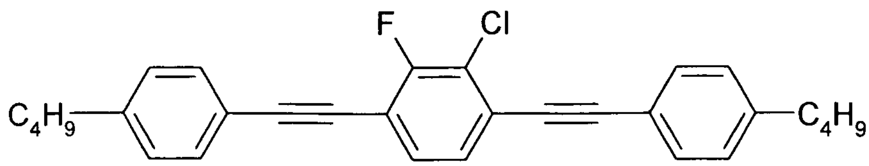

Die Titelverbindung wird analog zu Beispiel 1 hergestellt.

K 80 N 192 I

$$\Delta\varepsilon = -0{,}9$$

$$\Delta n = 0{,}426$$

$$\gamma_1 = 508\ mPa\cdot s$$

Synthesebeispiel 11:

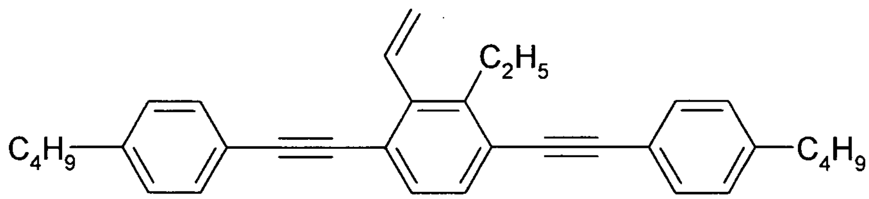

Die Titelverbindung wird analog zu Beispiel 1 hergestellt.

K 41 N 161 I

$$\Delta\varepsilon = 2{,}4$$

$$\Delta n = 0{,}437$$

Synthesebeispiel 12:

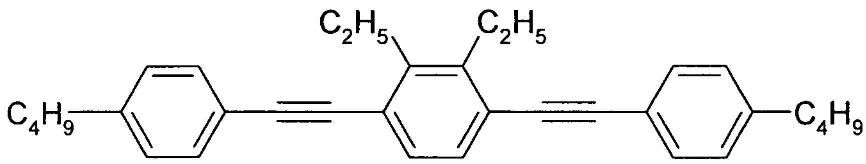

Die Titelverbindung wird analog zu Beispiel 1 hergestellt.

K 48 N 159 I

Synthesebeispiel 13:

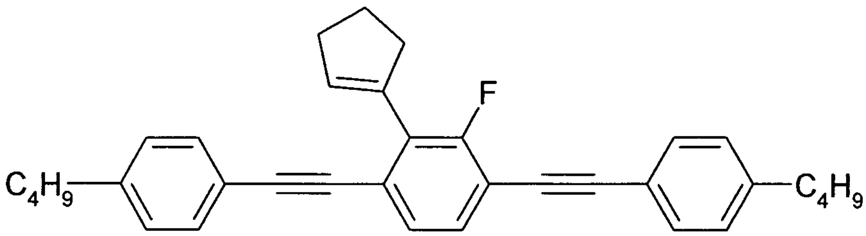

Die Titelverbindung wird analog zu Beispiel 1 hergestellt.

Tg -37 K 52 N 78 I

$$\Delta\varepsilon = 0{,}9$$

$$\Delta n = 0{,}339$$

$$\gamma_1 = 2219\ \mathrm{mPa \cdot s}$$

Synthesebeispiel 14:

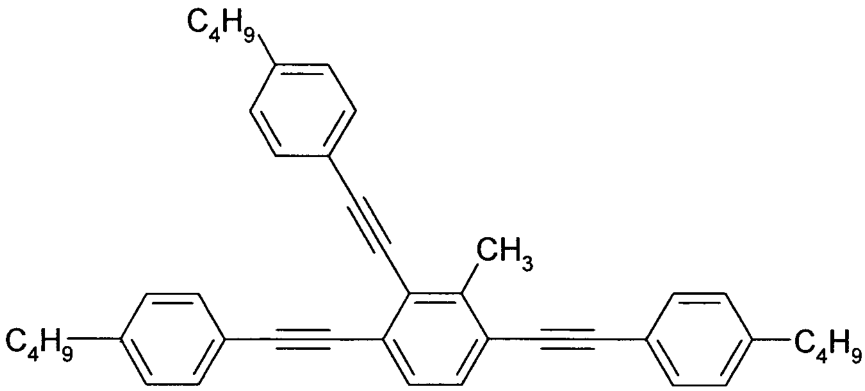

Die Titelverbindung wird analog zu Beispiel 1 hergestellt.

Tg -34 K 72 N (49) I

$$\Delta\varepsilon = 0,8$$

$$\Delta n = 0,302$$

$$\gamma_1 = 785 \text{ mPa·s}$$

Synthesebeispiel 15:

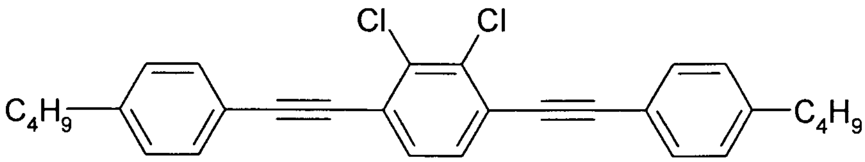

Die Titelverbindung wird analog zu Beispiel 1 hergestellt.

K 97 N 204 I

$$\Delta\varepsilon = -0,9$$

$$\Delta n = 0,421$$

$$\gamma_1 = 610 \text{ mPa·s}$$

Synthesebeispiel 16:

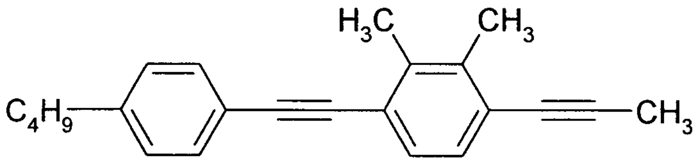

Die Titelverbindung wird analog zu Beispiel 1 hergestellt.

K 72 N (51) I

Mischungsbeispiel 1

Es wird ein Flüssigkristallmedium M-1 mit der Zusammensetzung und den Eigenschaften wie in der folgenden Tabelle angegeben hergestellt. Die Verbindung (1) (Nr. 15) stammt aus dem Synthesebeispiel 8.

| Zusammensetzung | | | Physikalische Eiqenschaften | |
|---|---|---|---|---|
| Verbindung | | | T(N,I) | = 96 °C |
| Nr. | Abkürzung | | | |
| 1 | BCH-3F.F | 10,8% | | |
| 2 | BCH-5F.F | 9,00 % | $\Delta$n (20°C, 589,3 nm) = | 0,124 |
| 3 | ECCP-30CF3 | 4,50 % | | |
| 4 | ECCP-50CF3 | 4,50 % | | |
| 5 | CBC-33F | 1,80 % | $\Delta\varepsilon$ (20°C, 1kHz) | = 4,9 |
| 6 | CBC-53F | 1,80 % | | |
| 7 | CBC-55F | 1,80% | $\gamma_1$ (20°C) | = 166 mPa·s |
| 8 | PCH-6F | 7,20% | | |
| 9 | PCH-7F | 5,40% | | |
| 10 | CCP-20CF3 | 7,20 % | | |
| 11 | CCP-30CF3 | 10,8% | | |
| 12 | CCP-40CF3 | 6,30% | | |
| 13 | CCP-50CF3 | 9,90% | | |
| 14 | PCH-5F | 9,00% | | |
| 15 | **(1)** | 10.0% | | |
| $\Sigma$ | | 100.0% | | |

Diese Mischung wird für Anwendungen im Mikrowellenbereich, insbesondere für Phasenschieber für 'phased array' Antennen verwendet.

Zum Vergleich wird ein Medium C-1 ohne die Komponente (1) aus den Verbindungen Nr. 1-14 des Mediums M-1 hergestellt, wobei die Verbindungen Nr. 1-14 in den gleichen relativen Mengen enthalten sind.

Tabelle: Eigenschaften der Mischungen M-1 und C-2 (Vergleich) bei 19 GHz (20°C)

| Mischung | $\varepsilon_{r,\parallel}$ | $\varepsilon_{r,\perp}$ | $\tau$ | tan $\delta_{\varepsilon,r,\parallel}$ | tan $\delta_{\varepsilon,r,\perp}$ | $\eta$ |
|---|---|---|---|---|---|---|
| M-1 | 2,57 | 2,33 | 0,097 | 0,0044 | 0,0130 | 7,41 |
| C-1 | 2,49 | 2,30 | 0,079 | 0,0048 | 0,0139 | 5,70 |

Die Steuerbarkeit $\tau$ und die Materialgüte $\eta$ sind gegenüber der Vergleichsmischung C-1 verbessert.

Weitere Kombinationen der Ausführungsformen und Varianten der Erfindung gemäß der Beschreibung ergeben

sich auch aus den folgenden Ansprüchen.

**Patentansprüche**

**1.** Verbindungen der Formel I

$$R^1\text{-}(A^1\text{-}Z^1)_m\text{—}A^2\text{—}\equiv\text{—}A^3\text{—}\equiv\text{—}(A^4)_k\text{—}(Z^5\text{-}A^5)_n\text{-}R^2 \qquad \mathrm{I}$$

worin

$A^3$

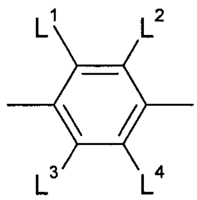

$L^1$, $L^2$ jeweils unabhängig voneinander verzweigtes oder unverzweigtes Alkyl, Alkenyl oder Alkinyl mit 1 bis 12 C-Atomen, worin unabhängig voneinander auch eine oder mehrere "-$CH_2$-"-Gruppen durch O ersetzt sein können, substituiertes oder unsubstituiertes Cycloalkyl, Cycloalkenyl, substituiertes oder unsubstituiertes Phenyl, substituiertes oder unsubstituiertes Arylethinyl, fluoriertes Alkyl oder Alkenyl, fluoriertes Alkyloxy oder Alkenyloxy, F, Cl, Br, CN, NCS oder $SF_5$
$L^3$, $L^4$ H,
$R^1$ und $R^2$ unabhängig voneinander einen halogenierten oder unsubstituierten Alkylrest mit 1 bis 15 C-Atomen, wobei in diesen Resten auch eine oder mehrere $CH_2$-Gruppen jeweils unabhängig voneinander durch -C≡C-, -CH=CH-, -CF=CF-, -CF=CH-, -CH=CF-, -(CO)- und -O- so ersetzt sein können, dass O-Atome nicht direkt miteinander verknüpft sind, F, Cl, Br, CN, $CF_3$, $OCF_3$, NCS oder $SF_5$, $R^2$ auch H,
$A^1$, $A^2$, $A^4$, $A^5$ jeweils unabhängig voneinander
a) 1,4-Phenylen, worin ein oder mehrere CH-Gruppen durch N ersetzt sein können,
b) trans-1,4-Cyclohexylen oder Cyclohexenylen, worin auch eine oder zwei nicht benachbarte $CH_2$-Gruppen durch -O- und/oder -S- ersetzt sein können, und worin H durch F ersetzt sein kann,
c) einen Rest der Formeln

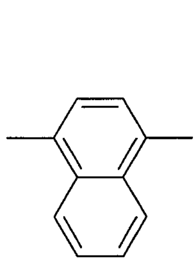

,

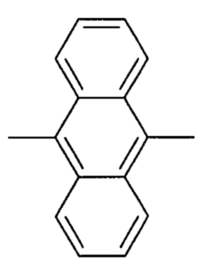

oder

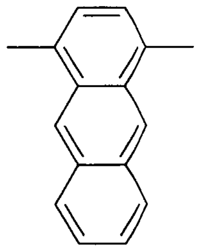,

oder

d) einen Rest aus der Gruppe 1,4-Bicyclo(2,2,2)-octylen, Cylcobut-1,3-diyl, Spiro[3.3]heptan-2,6-diyl, Thiophen-2,5-diyl, Thiophen-2,4-diyl, Furan-2,5-diyl, Furan-2,4-diyl,

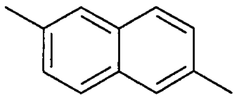

oder

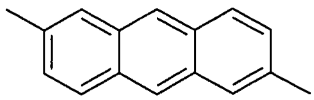,

und worin in den Gruppen a), b), c) und d)
auch ein oder mehrere H-Atome gegen Br, Cl, F, CN, -NCS, $SF_5$, $C_1$-$C_{10}$ Alkyl, $C_1$-$C_{10}$ Alkoxy oder eine ein- oder mehrfach fluorierte $C_1$-$C_{10}$ Alkyl- oder Alkoxygruppe ersetzt sein können,

k 0 oder 1,
$Z^1$, $Z^5$ unabhängig voneinander eine Einfachbindung, -C≡C-, -CH=CH-, $-CH_2O-$, -(CO)O-, $-CF_2O-$, $-CF_2CF_2-$, $-CH_2CF_2-$, $-CH_2CH_2-$, $-(CH_2)_4-$, -CH=CF- oder -CF=CF-, wobei asymmetrische Brücken nach beiden Seiten orientiert sein können,

wobei für k = 0 $Z^5$ eine Einfachbindung ist, und
m, n unabhängig voneinander 0, 1 oder 2 bedeuten.

2. Verbindungen nach Anspruch 1, **dadurch gekennzeichnet, dass** genau eine Gruppe aus $L^1$ und $L^2$ F bedeutet.

3. Verbindungen nach Anspruch 1 oder 2, **dadurch gekennzeichnet, dass**
$L^1$ und/oder $L^2$ unabhängig

einen halogenierten oder unsubstituierten Alkylrest mit 1 bis 12 C-Atomen, wobei in diesen Resten auch eine oder mehrere $CH_2$-Gruppen jeweils unabhängig voneinander durch -C≡C-, -CH=CH-, -CF=CF-, -CF=CH-, -CH=CF-, -(CO)O-, -O(CO)-, -(CO)-, -O- oder -S- so ersetzt sein können, dass O- oder S-Atome nicht direkt miteinander verknüpft sind,
optional durch Alkyl substituiertes Cycloalkyl mit 3 bis 6 C-Atomen, oder
optional durch Halogen, $C_{1-6}$-Alkyl oder $C_{1-6}$-Alkoxy substituiertes Phenyl

bedeuten.

4. Verbindungen nach einem oder mehreren der Ansprüche 1 bis 3, **dadurch gekennzeichnet, dass** m und n 0 sind.

5. Verbindungen nach einem oder mehreren der Ansprüche 1 bis 4, **dadurch gekennzeichnet, dass** k 1 ist.

6. Flüssigkristallmedium, **dadurch gekennzeichnet, dass** es eine oder mehrere Verbindungen der Formel I nach

einem oder mehreren der Ansprüche 1 bis 5 enthält.

7. Flüssigkristallmedium nach Anspruch 6, **dadurch gekennzeichnet, dass** es zusätzlich eine oder mehrere Verbindungen ausgewählt aus den Verbindungen der Formel II enthält:

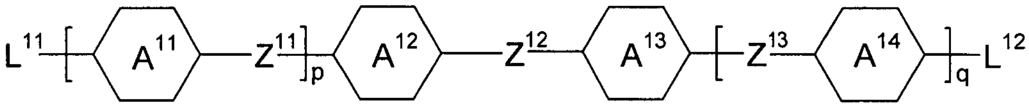 II

worin bedeuten:

$L^{11}$ $R^{11}$ oder $X^{11}$,
$L^{12}$ $R^{12}$ oder $X^{12}$,
$R^{11}$ und $R^{12}$ unabhängig voneinander unfluoriertes Alkyl oder unfluoriertes Alkoxy mit 1 bis 17 C-Atomen oder unfluoriertes Alkenyl, unfluoriertes Alkinyl, unfluoriertes Alkenyloxy, oder unfluoriertes Alkoxyalkyl mit 2 bis 15 C-Atomen,
$X^{11}$ und $X^{12}$ unabhängig voneinander F, Cl, Br, -CN, -NCS, -SCN, $-SF_5$, fluoriertes Alkyl oder fluoriertes Alkoxy mit 1 bis 7 C-Atomen oder fluoriertes Alkenyl, fluoriertes Alkenyloxy oder fluoriertes Alkoxyalkyl mit 2 bis 7 C-Atomen,
p, q unabhängig 0 oder 1,
$Z^{11}$ bis $Z^{13}$ unabhängig voneinander *trans*- -CH=CH-, *trans*- -CF=CF-, -C=C- oder eine Einfachbindung, und

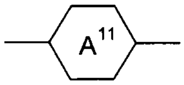

bis

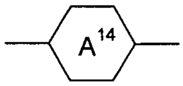

unabhängig voneinander

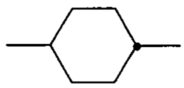 , 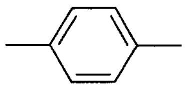 , 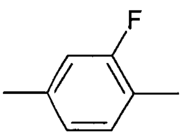 ,

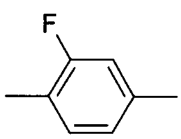 , 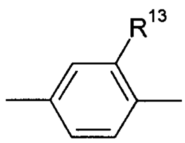 , 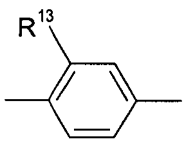 ,

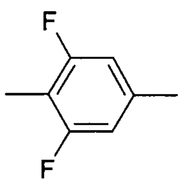 , 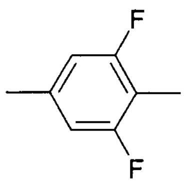 , 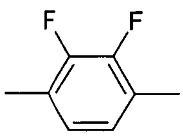 ,

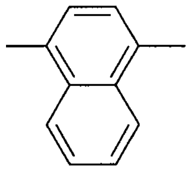

,

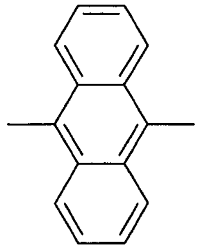

oder

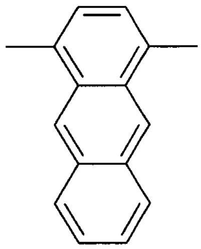

,

wobei $R^{13}$ Cl, Alkyl, $C_{1-5}$ Alkenyl oder $C_{3-6}$ Cycloalkyl bedeutet.

8. Flüssigkristallmedium nach Anspruch 6 oder 7, **dadurch gekennzeichnet, dass** die Konzentration der Verbindungen der Formel I im Medium im Bereich von insgesamt 5 % bis 95 % liegt.

9. Verwendung der Verbindungen der Formel I nach einem oder mehreren der Ansprüche 1 bis 5 in einem flüssigkristallinen Medium.

10. Verwendung der Verbindungen der Formel I nach einem oder mehreren der Ansprüche 1 bis 5 in einem Bauteil für die Hochfrequenztechnik.

11. Verfahren zur Herstellung eines Flüssigkristallmediums nach einem oder mehreren der Ansprüche 6 bis 8, **dadurch gekennzeichnet, dass** eine oder mehrere Verbindungen der Formel I mit einer oder mehreren weiteren Verbindungen und optional mit einem oder mehreren Additiven gemischt wird.

12. Bauteil für die Hochfrequenztechnik, **dadurch gekennzeichnet, dass** es ein Flüssigkristallmedium nach einem oder mehreren der Ansprüche 6 bis 8 enthält.

13. Bauteil nach Anspruch 12, **dadurch gekennzeichnet, dass** es sich um einen oder mehrere funktionell verbundene Phasenschieber handelt.

14. Verwendung eines Flüssigkristallmediums nach einem oder mehreren der Ansprüche 6 bis 8 in einem Bauteil für die Hochfrequenztechnik.

15. 'Phased array' Antenne, **dadurch gekennzeichnet**, das sie ein oder mehrere Bauteile nach Anspruch 12 oder 13 enthält.

## Claims

1. Compounds of the formula I

$$R^1\text{-}(A^1\text{-}Z^1)_m\text{—}A^2\text{—}\equiv\text{—}A^3\text{—}\equiv\text{—}(A^4)_k\text{—}(Z^5\text{-}A^5)_n\text{-}R^2 \qquad \mathrm{I}$$

in which

$A^3$ denotes

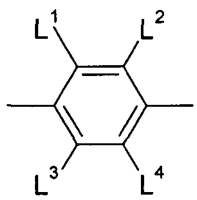

$L^1$, $L^2$ each, independently of one another, denote branched or unbranched alkyl, alkenyl or alkynyl having 1 to 12 C atoms, in which, in addition, one or more "$-CH_2-$" groups may be replaced, independently of one another, by O, or denote substituted or unsubstituted cycloalkyl, cycloalkenyl, substituted or unsubstituted phenyl, substituted or unsubstituted arylethynyl, fluorinated alkyl or alkenyl, fluorinated alkoxy or alkenyloxy, F, Cl, Br, CN, NCS or $SF_5$,
$L^3$, $L^4$ denote H,
$R^1$ and $R^2$, independently of one another, denote a halogenated or unsubstituted alkyl radical having 1 to 15 C atoms, where, in addition, one or more $CH_2$ groups in these radicals may each be replaced, independently of one another, by -C≡C-, -CH=CH-, -CF=CF-, -CF=CH-, -CH=CF-, -(CO)- and -O- in such a way that O atoms are not linked directly to one another, F, Cl, Br, CN, $CF_3$, $OCF_3$, NCS or $SF_5$, $R^2$ also denotes H,
$A^1$, $A^2$, $A^4$, $A^5$ each, independently of one another, denote
a) 1,4-phenylene, in which one or more CH groups may be replaced by N,
b) trans-1,4-cyclohexylene or cyclohexenylene, in which, in addition, one or two non-adjacent $CH_2$ groups may be replaced by -O- and/or -S-, and in which H may be replaced by F,
c) a radical of the formula

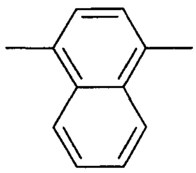

,

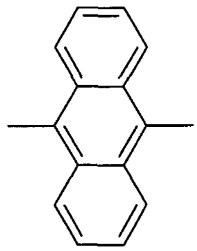

or

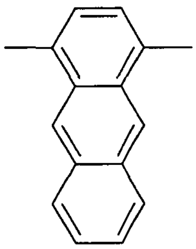

,

or
d) a radical from the group 1,4-bicyclo[2.2.2]octylene, cyclobutane-1,3-diyl, spiro[3.3]heptane-2,6-diyl, thiophene-2,5-diyl, thiophene-2,4-diyl, furan-2,5-diyl, furan-2,4-diyl,

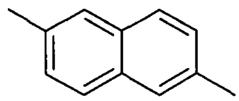

or

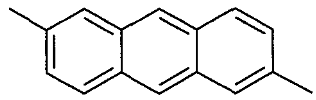
,

and in which, in groups a), b), c) and d), in addition one or more H atoms may be replaced by Br, Cl, F, CN, -NCS, $SF_5$, $C_1$-$C_{10}$ alkyl, $C_1$-$C_{10}$ alkoxy or a mono- or polyfluorinated $C_1$-$C_{10}$ alkyl or alkoxy group,

k denotes 0 or 1,
$Z^1$, $Z^5$, independently of one another, denote a single bond, -C≡C-, -CH=CH-, $-CH_2O-$ -(CO)O-, $-CF_2O-$, $-CF_2CF_2-$, $-CH_2CF_2-$, $-CH_2CH_2-$, $-(CH_2)_4-$, -CH=CF- or -CF=CF-, where asymmetrical bridges may be oriented to both sides, where, for k = 0, $Z^5$ is a single bond, and
m, n, independently of one another, denote 0, 1 or 2.

2. Compounds according to Claim 1, **characterised in that** precisely one group from $L^1$ and $L^2$ denotes F.

3. Compounds according to Claim 1 or 2, **characterised in that** $L^1$ and/or $L^2$ independently denote

a halogenated or unsubstituted alkyl radical having 1 to 12 C atoms, where, in addition, one or more $CH_2$ groups in these radicals may each be replaced, independently of one another, by -C≡C-, -CH=CH-, -CF=CF-, -CF=CH-, -CH=CF-, -(CO)O-, -O(CO)-, -(CO)-, -O- or -S- in such a way that O or S atoms are not linked directly to one another,
cycloalkyl having 3 to 6 C atoms which is optionally substituted by alkyl, or
phenyl which is optionally substituted by halogen, $C_{1-6}$-alkyl or $C_{1-6}$-alkoxy.

4. Compounds according to one or more of Claims 1 to 3, **characterised in that** m and n are 0.

5. Compounds according to one or more of Claims 1 to 4, **characterised in that** k is 1.

6. Liquid-crystal medium, **characterised in that** it comprises one or more compounds of the formula I according, to one or more of Claims 1 to 5.

7. Liquid-crystal medium according to Claim 6, **characterised in that** it additionally comprises one or more compounds selected from the compounds of the formula II:

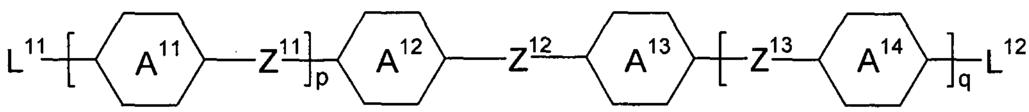
II

in which:

$L^{11}$ denotes $R^{11}$ or $X^{11}$,
$L^{12}$ denotes $R^{12}$ or $X^{12}$,
$R^{11}$ and $R^{12}$, independently of one another, denote unfluorinated alkyl or unfluorinated alkoxy having 1 to 17 C atoms or unfluorinated alkenyl, unfluorinated alkynyl, unfluorinated alkenyloxy or unfluorinated alkoxyalkyl having 2 to 15 C atoms,
$X^{11}$ and $X^{12}$, independently of one another, denote F, Cl, Br, -CN, -NCS, -SCN, $-SF_5$, fluorinated alkyl or fluorinated alkoxy having 1 to 7 C atoms or fluorinated alkenyl, fluorinated alkenyloxy or fluorinated alkoxyalkyl having 2 to 7 C atoms,

p, q independently denote 0 or 1,

$Z^{11}$ to $Z^{13}$, independently of one another, denote *trans*-CH=CH-, *trans*-CF=CF-, -C=C- or a single bond, and

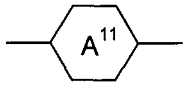

to

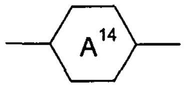

,

independently of one another, denote

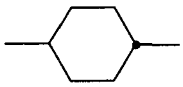

,

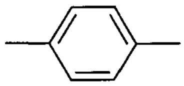

,

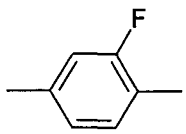

,

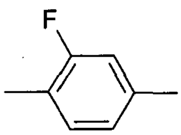

,

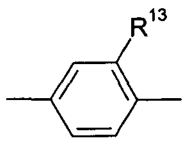

,

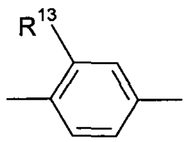

,

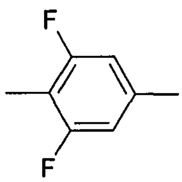

,

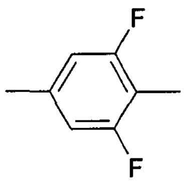

,

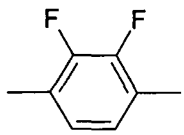

,

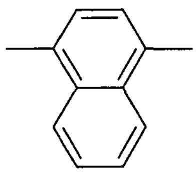

,

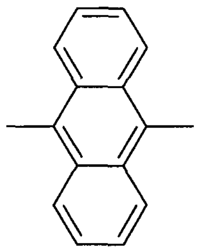

or

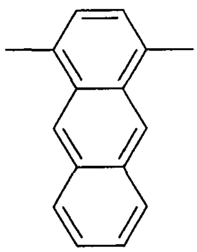

,

where $R^{13}$ denotes Cl, $C_{1-5}$ alkyl, $C_{1-5}$ alkenyl or $C_{3-6}$ cycloalkyl.

8. Liquid-crystal medium according to Claim 6 or 7, **characterised in that** the concentration of the compounds of the formula I in the medium is in the range from in total 5% to 95%.

9. Use of the compounds of the formula I according to one or more of Claims 1 to 5 in a liquid-crystalline medium.

10. Use of the compounds of the formula I according to one or more of Claims 1 to 5 in a component for high-frequency technology.

11. Process for the preparation of a liquid-crystal medium according to one or more of Claims 6 to 8, **characterised in that** one or more compounds of the formula I are mixed with one or more further compounds and optionally with one or more additives.

12. Component for high-frequency technology, **characterised in that** it contains a liquid-crystal medium according to one or more of Claims 6 to 8.

13. Component according to Claim 12, **characterised in that** it is one or more functionally connected phase shifters.

14. Use of a liquid-crystal medium according to one or more of Claims 6 to 8 in a component for high-frequency technology.

15. 'Phased array' antenna, **characterised in that** it contains one or more components according to Claim 12 or 13.

## Revendications

1. Composés de la formule I :

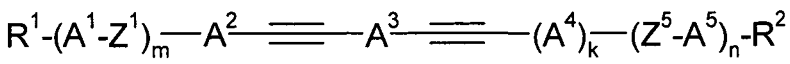

I

dans laquelle :

$A^3$ représente

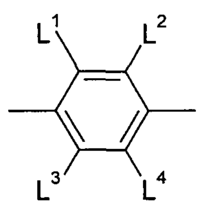

$L^1$, $L^2$ représentent chacun, indépendamment l'un de l'autre, alkyle, alkényle ou alkynyle ramifié ou non ramifié comportant 1 à 12 atome(s) de C, où, en outre, un ou plusieurs groupe(s) "$-CH_2-$" peut/ peuvent être remplacé(s), indépendamment les uns des autres, par O, ou représentent chacun cycloalkyle, cycloalkényle substitué ou non substitué, phényle substitué ou non substitué, aryléthynyle substitué ou non substitué, alkyle ou alkényle fluoré, alcoxy ou alkényloxy fluoré, F, Cl, Br, CN, NCS ou SFs,
$L^3$, $L^4$ représentent H,
$R^1$ et $R^2$ représentent, indépendamment l'un de l'autre, un radical alkyle halogéné ou non substitué comportant 1 à 15 atome(s) de C, où, en outre, un ou plusieurs groupe(s) $CH_2$ dans ces radicaux peut/peuvent chacun être remplacé(s), indépendamment les uns des autres, par -C≡C-, -CH=CH-, -CF=CF-, -CF=CH-, -CH=CF-, -(CO)- et -O- de telle sorte que des atomes de O ne soient pas liés directement les uns aux autres, F, Cl, Br, CN, $CF_3$, $OCF_3$, NCS ou $SF_5$, $R^2$ représente également H,
$A^1$, $A^2$, $A^4$, $A^5$ représentent chacun, indépendamment les uns des autres,
a) 1,4-phénylène, où un ou plusieurs groupe(s) CH peut/peuvent être remplacé(s) par N,

b) trans-1,4-cyclohexylène ou cyclohexénylène, où, en outre, un ou deux groupe(s) $CH_2$ non adjacents peut/peuvent être remplacé(s) par -O- et/ou -S-, et où H peut être remplacé par F,
c) un radical de la formule :

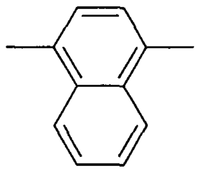

,

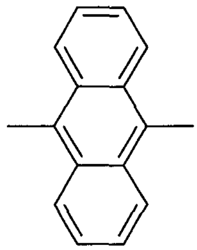

ou

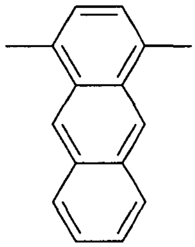

,

ou

d) un radical pris parmi le groupe 1,4-bicyclo[2.2.2]octylène, cyclobutane-1,3-diyle, spiro[3.3]heptane-2,6-diyle, thiophène-2,5-diyle, thiophène-2,4-diyle, furan-2,5-diyle, furan-2,4-diyle,

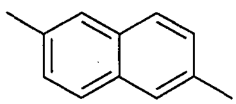

ou

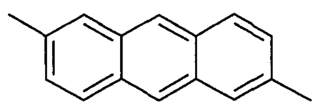

,

et où, dans les groupes a), b), c) et d), en outre, un ou plusieurs atome(s) de H peut/peuvent être remplacé(s) par Br, Cl, F, CN, -NCS, $SF_5$, $C_1$-$C_{10}$ alkyle, $C_1$-$C_{10}$ alcoxy ou un groupe $C_1$-$C_{10}$ alkyle ou alcoxy monofluoré ou polyfluoré,
k représente 0 ou 1,
$Z^1$, $Z^5$ représentent, indépendamment l'un de l'autre, une liaison simple, -C≡C-, -CH=CH-, $-CH_2O-$, -(CO)O-, $-CF_2O-$, $-CF_2CF_2-$, $-CH_2CF_2-$, $-CH_2CH_2-$, $-(CH_2)_4-$, -CH=CF- ou -CF=CF-, où des ponts asymétriques peuvent

être orientés sur les deux côtés, où, pour k = 0, $Z^5$ est une liaison simple, et
m, n représentent, indépendamment l'un de l'autre, 0, 1 ou 2.

2. Composés selon la revendication 1, **caractérisés en ce que** précisément un groupe pris parmi $L^1$ et $L^2$ représente F.

3. Composés selon la revendication 1 ou 2, **caractérisés en ce que** $L^1$ et/ou $L^2$ représente(nt), de manière indépendante, un radical alkyle halogéné ou non substitué comportant 1 à 12 atome(s) de C, où, en outre, un ou plusieurs groupe(s) $CH_2$ dans ces radicaux peut/peuvent chacun être remplacé(s), indépendamment les uns des autres, par -C≡C-, -CH=CH-, -CF=CF-, -CF=CH-, -CH=CF-, -(CO)O-, -O(CO)-, -(CO)-, -O- ou -S- de telle sorte que des atomes de O ou de S ne soient pas liés directement les uns aux autres,

cycloalkyle comportant 3 à 6 atomes de C, lequel est en option substitué par alkyle, ou
phényle, lequel est en option substitué par halogène, $C_{1-6}$-alkyle ou $C_{1-6}$-alcoxy.

4. Composés selon une ou plusieurs des revendications 1 à 3, **caractérisés en ce que** m et n valent 0.

5. Composés selon une ou plusieurs des revendications 1 à 4, **caractérisés en ce que** k vaut 1.

6. Milieu cristallin liquide, **caractérisé en ce qu'**il comprend un ou plusieurs composé(s) de la formule I selon une ou plusieurs des revendications 1 à 5.

7. Milieu cristallin liquide selon la revendication 6, **caractérisé en ce qu'**il comprend de façon additionnelle un ou plusieurs composé(s) choisi(s) parmi les composés de la formule II :

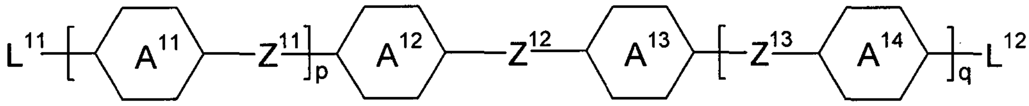

II

dans laquelle :

$L^{11}$ représente $R^{11}$ ou $X^{11}$,
$L^{12}$ représente $R^{12}$ ou $X^{12}$,
$R^{11}$ et $R^{12}$ représentent, indépendamment l'un de l'autre, alkyle non fluoré ou alcoxy non fluoré comportant 1 à 17 atome(s) de C ou alkényle non fluoré, alkynyle non fluoré, alkényloxy non fluoré ou alcoxyalkyle non fluoré comportant 2 à 15 atomes de C,
$X^{11}$ et $X^{12}$ représentent, indépendamment l'un de l'autre, F, Cl, Br, -CN, -NCS, -SCN, $-SF_5$, alkyle fluoré ou alcoxy fluoré comportant 1 à 7 atome(s) de C ou alkényle fluoré, alkényloxy fluoré ou alcoxyalkyle fluoré comportant 2 à 7 atomes de C,
p, q représentent de manière indépendante 0 ou 1,
$Z^{11}$ à $Z^{13}$ représentent, indépendamment les uns des autres, *trans*-CH=CH-, *trans*-CF=CF-, -C=C- ou une liaison simple, et

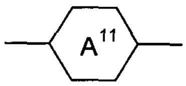

à

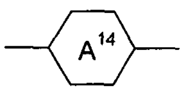

,

représentent, indépendamment les uns des autres,

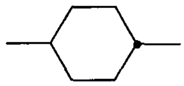 , 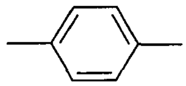 , 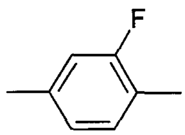 ,

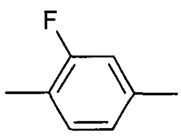 , 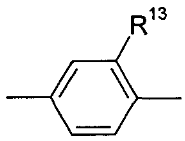 , 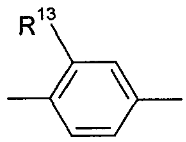 ,

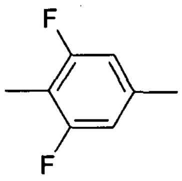 , 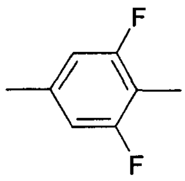 , 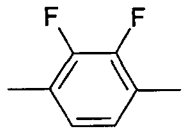 ,

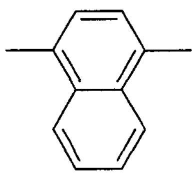 , 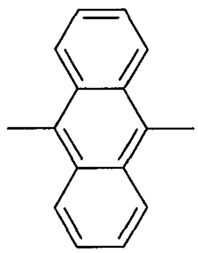

ou

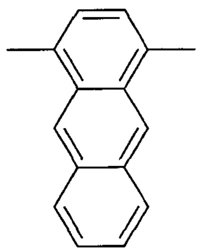 ,

où $R^{13}$ représente Cl, $C_{1-5}$ alkyle, $C_{1-5}$ alkényle ou $C_{3-6}$ cycloalkyle.

**8.** Milieu cristallin liquide selon la revendication 6 ou 7, **caractérisé en ce que** la concentration des composés de la formule I dans le milieu est dans la plage de 5% à 95% au total.

**9.** Utilisation des composés de la formule I selon une ou plusieurs des revendications 1 à 5 dans un milieu cristallin liquide.

**10.** Utilisation des composés de la formule I selon une ou plusieurs des revendications 1 à 5 dans un composant pour une technologie haute fréquence.

**11.** Procédé pour la préparation d'un milieu cristallin liquide selon une ou plusieurs des revendications 6 à 8, **caractérisé en ce qu'**un ou plusieurs composé(s) de la formule I est/sont mélangé(s) avec un ou plusieurs autre(s) composé(s) et en option, avec un ou plusieurs additif(s).

**12.** Composant pour une technologie haute fréquence, **caractérisé en ce qu'**il contient un milieu cristallin liquide selon une ou plusieurs des revendications 6 à 8.

**13.** Composant selon la revendication 12, **caractérisé en ce qu'**il s'agit d'un ou de plusieurs déphaseur(s) connecté(s)

fonctionnellement.

14. Utilisation d'un milieu cristallin liquide selon une ou plusieurs des revendications 6 à 8 dans un composant pour une technologie haute fréquence.

15. Antenne 'réseau à commande de phase', **caractérisée en ce qu'**elle contient un ou plusieurs composant(s) selon la revendication 12 ou 13.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0968988 A1
- DE 19907941 A1
- DE 10120024 A1
- JP 08012599 A
- DE 102004029429 A
- JP 2005120208 A
- EP 0581272 A1


### In der Beschreibung aufgeführte Nicht-Patentliteratur


- **A. GAEBLER ; A. MOESSINGER ; F. GOELDEN et al.** Liquid Crystal-Reconfigurable Antenna Concepts for Space Applications at Microwave and Millimeter Waves. *International Journal of Antennas and Propagation,* 2009, vol. 2009, 7
- Cavity Perturbation Method for Characterization of Liquid Crystals up to 35 GHz. **A. PENIRSCHKE ; S. MÜLLER ; P. SCHEELE ; C. WEIL ; M. WITTEK ; C. HOCK ; R. JAKOBY.** 34th European Microwave Conference - Amsterdam, 545-548 beschreibt unter anderen die Eigenschaften der bekannten, flüssigkristallinen Einzelsubstanz K15. Merck KGaA
- **S.-T. WU ; C.-S. HSU ; K.-F. SHYU.** *Appl. Phys. Lett.,* 1999, vol. 74 (3), 344-346
- **C. S. HSU ; K. F. SHYU ; Y.Y. CHUANG ; S.-T. WU LIQ.** *Cryst.,* 2000, vol. 27 (2), 283-287
- Merck Liquid Crystals, Physical Properties of Liquid Crystals. Stand. Merck KGaA, November 1997
- **A. PENIRSCHKE et al.** Cavity Perturbation Method for Characterization of Liquid Crystals up to 35GHz. *34th European Microwave Conference - Amsterdam,* 545-548
- Direct Simulation of Material Permittivites ... **A. GAEBLER et al.** 12MTC 2009 - International Instrumentation and Measurement Technology Conference. IEEE, 2009, 463-467
- **A. PENIRSCHKE et al.** *34th European Microwave Conference - Amsterdam,* 545-548